Europäisches Patentamt

European Patent Office

Office européen des brevets

(11) Publication number: **0 332 424**
A2

## (12) EUROPEAN PATENT APPLICATION

(21) Application number: 89302312.7

(22) Date of filing: 08.03.89

(51) Int. Cl.⁴: **C 12 N 15/00**
C 12 N 5/00, A 61 K 39/395

(30) Priority: 09.03.88 US 165856   17.11.88 US 272577

(43) Date of publication of application:
13.09.89 Bulletin 89/37

(84) Designated Contracting States:
AT BE CH DE ES FR GB GR IT LI LU NL SE

(71) Applicant: HYBRITECH INCORPORATED
11095 Torreyana Road
San Diego California 92126 (US)

(72) Inventor: Beidler, Catherine Brautigam
12385 Paseo Colina
Poway California 92064 (US)

Johnson, Mary Jacqueline
16231 El Camino Real
Rancho Sante Fe California 92067 (US)

Ludwig, James Richard
2058 Seca Street
El Cajon California 92020 (US)

Carlo, Dennis J.
1176 Los Pinos
Rancho Sante Fe California (US)

David, Gary Samuel
9477 Poole Street
La Jolla California 92037 (US)

(74) Representative: Hudson, Christopher Mark et al
Erl Wood Manor
Windlesham Surrey GU20 6PH (GB)

Claims for the following Contracting States: ES + GR
The microorganism(s) has (have) been deposited with ATCC 9620/67637/67638/67639/67640 under numbers NRRL B - 18432 NRRL B - 18433

(54) Chimeric antibodies directed against human carcinoembryonic antigen.

(57) The present invention discloses novel chimeric monoclonal antibodies, directed against human carcinoembryonic antigen, having antigen-specific variable regions of defined amino acid sequences. DNA constructs for the light and heavy chain variable regions comprising the novel antibodies of the invention are provided. Eukaryotic host cells capable of expression of the chimeric antibodies and comprising the novel chimeric antibody-encoding DNA constructs are also provided.

EP 0 332 424 A2

**Description**

## CHIMERIC ANTIBODIES DIRECTED AGAINST HUMAN CARCINOEMBRYONIC ANTIGEN

The present invention relates to monoclonal antibodies directed against human carcinoembryonic antigen. More particularly, it relates to novel chimeric monoclonal antibodies directed against human carcinoembryonic antigen, and DNA constructs encoding for such antibodies, for in vitro and in vivo application.

Monoclonal antibodies are becoming increasingly important for both in vitro application in immunoassays and for the in vivo diagnosis and treatment of disease. Monoclonal antibodies which are directed against human carcinoembryonic antigen ("CEA") are particularly useful for the in vivo imaging and treatment of tumors associated with certain carcinomas, including colorectal and breast carcinomas. Depending upon the clinical application, these monoclonal antibodies are generally conjugated with radionuclides, drugs or toxins.

Most available monoclonal antibodies, however, are derived from murine, i.e., mouse, hybridomas. The in vitro application of murine antibodies in immunoassays presents potential problems associated with false positive results which are attributable to the reaction of serum components with murine immunoglobulins. More importantly though, the in vivo application of murine antibodies in human medicine is often limited due to their inherent immunogenicity. The administration of murine antibodies will, in many patients, induce an immune response which results in a gradual decline in the efficacy of the antibodies during multiple dose regimens. This decrease in efficacy is attributable, at least in part, to the rapid clearance from circulation or alteration of pharmacokinetic properties of murine antibodies by the patient's immune response. The immunogenicity associated with murine monoclonal antibodies, therefore, precludes multiple dose administrations over an extended period of time and substantially impacts their potential clinical value. It has been suggested that the use of human monoclonal antibodies for clinical application would overcome the limitations associated with the use of murine monoclonal antibodies. However, human monoclonal antibodies having the desired specificity and affinity for tumor-associated antigens, such as human carcinoembryonic antigen, are technically difficult to prepare.

Chimeric antibodies, in which the binding or variable regions of antibodies derived from one species are combined with the constant regions of antibodies derived from a different species, have been constructed by recombinant DNA methodology. Chimeric antibodies are described, for example, in European Patent Publication 173494; Shaw, et al., J. Immun., 138:4534 (1987), Sun, L.K. et al., Proc. Natl. Acad. Sci. USA, 84:214-218 (1987); Neuberger, M.S. et al., Nature, 314:268 (1985), Boulianne, G.L. et al., Nature, 312:643-646 (1984); and Morrison, S.L. et al., Proc. Natl. Acad. Sci. USA, 81:6851-6855 (1984). Typically, the variable region of a murine antibody is joined with the constant region of a human antibody. It is expected that as such chimeric antibodies are largely human in composition, they will be substantially less immunogenic than murine antibodies. Accordingly, chimeric monoclonal antibodies are highly desirable for in vivo application.

While the general concept of chimeric antibodies has been described, there exists a need for the development of novel chimeric monoclonal antibodies having specificity for predetermined antigens of interest, particularly human carcinoembryonic antigen, and variable regions of defined amino acid sequences. Further, there exists a need for the development of DNA constructs having defined DNA coding sequences for light and heavy chain variable regions which comprise novel chimeric antibodies, and which are capable of expressing these novel chimeric proteins in eukayotic cells. The present invention meets these needs.

For purposes of the present invention, as disclosed and claimed herein, the following terms are abbreviated as indicated below:

| Amino Acid | Three Letter Abbreviation |
|---|---|
| Alanine | Ala |
| Arginine | Arg |
| Asparagine | Asn |
| Aspartic acid | Asp |
| Cysteine | Cys |
| Glutamic Acid | Glu |
| Glutamine | Gln |
| Glycine | Gly |
| Histidine | His |
| Isoleucine | Ile |
| Leucine | Leu |

| Amino Acid | Three Letter Abbreviation |
|---|---|
| Lysine | Lys |
| Methionine | Met |
| Phenylalanine | Phe |
| Proline | Pro |
| Serine | Ser |
| Threonine | Thr |
| Tryptophan | Trp |
| Tyrosine | Tyr |
| Valine | Val |

| Nucleic Acid | One Letter Abbreviation |
|---|---|
| Deoxyadenyl | A |
| Deoxyguanyl | G |
| Deoxycytidyl | C |
| Thymidyl | T |

The present invention, in one aspect, provides novel chimeric monoclonal antibodies directed against human carcinoembryonic antigen ("CEA"). In the context of the present invention, the term "chimeric antibody" refers to an antibody comprising an antigen-specific variable region derived from a first mammalian species and a constant region derived from a second and different mammalian species. Accordingly, the chimeric antibodies of the invention are the product of fused genes encoding for a variable region having specificity for CEA and a constant region having predetermined structural and physiological properties not naturally associated with the variable region.

As used herein, the term "variable region" refers to the region of light and heavy chain antibody molecules which is capable of binding CEA. The amino acid sequence of the variable region will vary depending upon the antigenic determinant to which it binds and the manner in which it recognizes that antigenic determinant. This diversity is related to the exon sequences in the genes which encode for the variable region.

The term "constant region", as used herein, refers to the region of light and heavy chain antibody molecules which provides structural stability and other biological functions but is not involved with binding CEA. The amino acid sequence and corresponding exon sequences in the genes of the constant region will be dependent upon the species from which it is derived; however, variations in the amino acid sequence will be relatively limited for particular constant regions within a species.

In another aspect, the present invention provides novel DNA constructs for chimeric polypeptides which comprise light and heavy chains of chimeric antibodies directed against CEA. Accordingly, the DNA constructs of the invention each comprise a first DNA sequence which encodes for the variable region of the chimeric polypeptide. For purposes of convenience, only the coding strand of the double stranded DNA is presented herein. The DNA constructs further comprise a second DNA sequence coding for the constant region of the chimeric polypeptide.

In accordance with the invention, DNA constructs for the light chains of chimeric antibodies directed against CEA comprise a first DNA sequence encoding for a light chain variable region which is substantially the same as:

GAC - ATT - GTG - ATG - ACC - CAG - TCT - CAA - AAA
TTC - ATG - TCC - ACA - TCA - GTA - GGA - GAC - AGG
GTC - AGC - ATC - ACC - TGC - AAG - GCC - AGT - CAG
AAT - GTT - CGT - ACT - GCT - GTT - GCC - TGG - TAT
CAA - CAG - AAA - CCA - GGG - CAG - TCT - CCT - AAA
GCA - CTG - ATT - TAC - TTG - GCA - TCC - AAC - CGG
TAC - ACT - GGA - GTC - CCT - GAT - CGC - TTC - ACA
GGC - AGT - GGA - TCT - GGG - ACA - GAT - TTC - ACT
CTC - ACC - ATT - ACC - AAT - GTG - CAA - TCT - GAA
GAC - CTG - GCA - GAT - TAT - TTC - TGT - CTG - CAA
CAT - TGG - AAT - TAT - CCG - CTC - ACG - TTC - GGT
GCT - GGG - ACC - AAG - CTG - GAG - CTG - AAA - CGG.

Also in accordance with the invention, DNA constructs for heavy chains of chimeric antibodies directed against CEA comprise a first DNA sequence encoding for a heavy chain variable region which is substantially the same as:

GAT - GTG - CAG - CTG - GTG - GAG - TCT - GGG - GGA
GGC - TTA - GTG - CAG - CCT - GGA - GGG - TCC - CGG
AAA - CTC - TCC - TGT - GCA - GCC - TCT - GGA - TTC
ACT - TTC - AGT - AAC - TTT - GGA - ATG - CAC - TGG
ATT - CGT - CAG - GCT - CCA - GAG - AAG - GGA - CTG
GAG - TGG - GTC - GCA - TAC - ATT - AGT - GGT - GGC
AGT - AGT - ACC - ATC - TAC - TAT - GCA - GAC - ACA
GTG - AAG - GGC - CGA - TTC - ACC - ATC - TCC - AGA
GAC - AAT - CCC - AGG - AAC - ACC - CTC - TTC - CTG
CAA - ATG - ACC - AGT - CTA - AGG - TCT - GAG - GAC
ACG - GCC - ATG - TTT - TAC - TGT - GCA - AGA - GAT
TAC - TAC - GCT - AAC - AAC - TAC - TGG - TAC - TTC
GAT - GTC - TGG - GGC - GCA - GGG - ACC - ACG - GTC
ACC - GTC - TCC - TCA - GCC.

Preferably, the first DNA coding sequences for the light and heavy chain variable regions further comprise a DNA sequence coding for a leader peptide for expression of these polypeptides by eukaryotic host cells. For purposes of convenience, only the coding strand of the double stranded DNA is presented herein. The DNA sequence encoding for the leader peptide of the variable light chain polypeptides is, preferably, substantially the same as:

ATG - GAG - TTT - CAG - ACC - CAG - GTC - TTT - GTA
TTC - GTG - TTG - CTC - TGG - TTG - TCT - GGT - GTT
GAT - GGA.

For expression of the variable heavy chain polypeptides, the DNA sequence of the leader peptide is, preferably, substantially the same as:

ATG - GAC - TCC - AGG - CTC - AAT - TTA - GTT - TTC - CTT
GTC - CTT - ATT - TTA - AAA - GGT - GTC - CAG - TGT.

The disclosed DNA sequences for the leader peptides include a translational start signal, i.e., a DNA sequence that initiates translation of functional polypeptides. Those skilled in the art will recognize that, as the leader peptide does not function in the binding of CEA, DNA sequences encoding for alternative eukaryotic leader

4

peptides may be suitably utilized in the invention. Accordingly, the present invention is not limited to the use of any particular eukaryotic leader peptide.

Further, it will be appreciated by those skilled in the art that the first DNA coding sequences comprising the DNA constructs of the invention may be modified, for example, by site-directed mutagenesis to provide DNA constructs which are substantially equivalent. These modified DNA coding sequences are included in the invention provided they are capable of being translated into substantially the same chimeric polypeptides as described herein. The use of site-directed mutagenesis may, in certain cases, modify the affinity of the resulting chimeric polypeptides for CEA.

The first DNA coding sequences of the DNA constructs of the present invention are preferably derived from the genomic DNA of a murine hybridome expressing monoclonal antibody directed against CEA. The murine hybridoma, designated as CEM 231.6.7, expressing antibody having the desired specificity and affinity for CEA, is particularly preferred for use. Murine hybridoma CEM 231.6.7 was deposited on January 7, 1988 with the American Type Culture Collection, Rockville, Maryland, under the accession number ATCC HB9620. However, the variable light and heavy chain regions may be derived from other mammalian species, e.g., lapine, caprine, equine, bovine, and non-human primates, provided the DNA sequences, and resulting amino acid sequences upon translation, are substantially equivalent to the sequences disclosed by the invention.

Genomic DNA for use in the invention can be obtained and cloned by conventional techniques and in a variety of ways. Such techniques are described in Basic Methods in Molecular Biology, edited by L.G. Davis, M.D. Dibner and J.F. Battey, Elsevier, New York (1986); Feder, J., et al., Am. J. Hum. Genetics, 37:635-649 (1985); and Steffer, D. and Weinberg, R.A., Cell, 15:1003-1010 (1978). For example, hybridoma cellular DNA may be isolated by standard procedures, the genomic DNA fragmented into restriction fragments by restriction endonucleases, and the resulting fragments cloned into suitable recombinant DNA cloning vectors and screened with radiolabeled or enzymatically labeled probes for the presence of the DNA sequences disclosed herein. As used herein, the term "restriction fragment" refers to any linear DNA sequence generated by the action of one or more restriction endonuclease enzymes. The term "recombinant DNA cloning vector", as used herein, refers to any autonomously replicating agent, including but not limited to, plasmids and phages, comprising a DNA molecule to which one or more additional DNA segments can be or have been added. The DNA sequences obtained from genomic DNA may also include intervening sequences or introns which do not code for polypeptides; these sequences can be subsequently modified by nucleotide deletion or substitution by standard procedures. See, for example, Kramer, W. et al., Nucleic Acids Res., 12:9441 (1984); and Kunkel, T.A., Proc. Nat. Acad. Sci., USA, 82:488 (1985).

. The first DNA sequences of the DNA constructs of the invention, encoding for polypeptides which are variable light and heavy regions of chimeric antibodies, can also be obtained from cDNA. Procedures for obtaining and cloning cDNA are well known and described by Okayama, H. and Berg, P., Mol. Cell Biol., 2:161 (1982); and Gubler, U. and Hoffman, B.J., Gene, 25:263 (1983). Accordingly, cDNA can be cloned by standard procedures and the resulting clones screened with a suitable probe for cDNA coding for the variable regions defined herein. After the desired clones have been isolated, the cDNA may be manipulated in essentially the same manner as genomic DNA.

Alternatively, the first DNA sequences, containing the requisite genetic information for variable light and heavy chain region specificity for CEA, may be synthetically prepared using conventional procedures. Techniques for synthesizing DNA sequences are described by Sinha, N.D. et al. Nucleic Acid Res., 12:4359 (1984); and Beaucage, S.L. and Caruthers, M.H., Tetrahedron Letters, 20:1859 (1981). The first DNA sequences encoding for light and heavy chain variable regions may, therefore, be synthesized from nucleotide monomers using conventional DNA synthesis technology to yield a DNA coding sequence capable of being translated into substantially the same polypeptides as described herein. This synthetic DNA sequence does not need to be identical to the cloned gene if degenerate codons are substituted for the original codons provided they code for the same amino acid upon translation.

The second DNA sequences of the DNA constructs of the invention, encoding for constant light and heavy chain regions of chimeric antibodies, can be cloned from genomic DNA and cDNA, or prepared synthetically. Preferably, the second DNA sequences encoding for constant region polypeptides are derived from human lymphocytes, e.g. human peripheral blood lymphocytes. The use of DNA sequences coding for human constant regions is expected to result in the production of chimeric light and heavy chain polypeptides which minimize immunogenicity. Particularly preferred for use are the DNA sequences derived from the human light (kappa) chain and the human heavy (gamma or other classes; and the various isotypes or allotypes thereof) chain genes, particularly the gamma-1 gene, described by Heiter, P.A. et al., Cell 22:197-207 (1980) and Takahashi, N. et al., Cell, 29:671-679 (1982). However, the present invention is not limited to particular second DNA sequences encoding for human constant regions for light and heavy chain chimeric polypeptides. Additionally, those skilled in the art will appreciate that, provided the species selected is different than the species from which the variable region is derived, the constant region genes may be derived from other mammalian species. For example, the constant region may be derived from species such as lapine, caprine, bovine, equine, porcine and non-human primates, depending upon whether the antibody will have in vitro or in vivo application.

The recombinant DNA techniques necessary to prepare the chimeric DNA constructs of the invention, and incorporate these constructs into appropriate recombinant DNA cloning vectors and recombinant DNA expression vectors, are now well known in the art and described by numerous references. See, e.g., Eukarytic Viral Vectors, edited by Y. Gluzman, Cold Spring Harbor Laboratories Publications, Cold Spring Harbor, New York (1982); Eukaryotic Transcription, edited by Y. Gluzman, Cold Spring Harbor, New York (1985); Sequence Specificity in Transcription & Translation, edited by R. Calendar and L. Gold, Allan R. Liss, Inc., New York, (1985); Maximizing Gene Expression, edited by W. Reznikoff and L. Gold, Butterworths, New York (1986); and Mammalian Cell Technology, edited by W.G. Thilly, Butterworths, New York (1986). As used herein, the term "recombinant DNA expression vector" refers to any recombinant DNA cloning vector which includes a promoter sequence for directing transcription of DNA into RNA, and appropriate regulatory sequences to initiate and terminate translation of RNA into polypeptides.

In accordance with the invention, the DNA constructs encoding for light and heavy chain chimeric polypeptides are introduced into appropriate eukaryotic host cells as part of an expression vector. These constructs can be contained on a single eukaryotic expression vector or maintained separately, with separate expression vectors each comprising a single chimeric gene construct. For expression of the chimeric polypeptides, however, it will be necessary to include transcriptional and translational regulatory sequences which are functional in the selected eukaryotic host cells. Accordingly, the chimeric genes can be isolated on large DNA fragments containing 5' and 3' untranslated regions as well as intronic sequences and comprising homologous regulatory regions, e.g., promoters, enhancers, and transcriptional terminators and polyadenylate addition sites ("poly A sites"), all of which function within eukaryotic host cells. As used herein, the terms "promoter" and "enhancer" refer to DNA sequences that direct transcription of DNA into RNA, and sequences that enhance transcription of DNA into RNA, respectively. The term "transcriptional terminator," as used herein, refers to a DNA sequence that terminates transcription of DNA into RNA. The term "poly A site" refers to a DNA sequence which designates location of a poly-A tail sequence attachment. Alternatively, the chimeric genes can be recombined with a variety of heterologous regulatory regions such as the well known SV40 and Herpes TK viral sequences, which contain viral promoters, enhancers, transcriptional terminators, and poly A sites. The chimeric gene constructs can also be combined with synthetic regulatory elements provided that these elements can function in eukaryotic host cells and are properly fused to the chimeric genes. cDNA clones or synthesized genes can also be combined with either homologous or heterologous regulatory sequences in order to be expressed as polypeptides. Skilled artisans will appreciate, therefore, that homologous, heterologous or synthetic regulatory regions may be used interchangeably for purposes of expression of the chimeric genes of the present invention.

A wide variety of recombinant expression vectors are well known and may be used in the invention. Preferably, the pSV2-type vectors are utilized. The pSV2-type vectors comprise segments of the SV40 genome that constitute a defined eukaryotic transcription unit and transform mammalian and other eukaryotic host cells by integrating into the host cell chromosomal DNA. A variety of plasmid pSV2-type vectors have been constructed (see, Eukaryotic Viral Vectors, edited by Y. Gluzman, Cold Spring Harbor Laboratory Publications, Cold Spring Harbor, New York, (1982)), such as plasmids pSV2-gpt, pSV2-neo, pSV2-dhfr, and pSV2-β-globin, in which the SV40 promoter directs transcription of an inserted gene. These vectors are available either from the American Type Culture Collection (ATCC) in Rockville, Maryland or from the Northern Regional Laboratory (NRRL) in Peoria, Illinois. Alternatively, expression vectors may be selected which can be maintained episomally, i.e., extrachromasomally, such as bovine papilloma virally-based expression vectors and Epstein Barr virus expression vectors. (See Eukaryotic Viral Vectors, edited by Y. Gluzman, Cold Spring Harbor Laboratories Publications, Cold Spring Harbor, New York (1982); Papillomaviruses, edited by P.M. Howley and T.R. Broker, Alan R. Liss, Inc., New York (1985); and Sugden, B. et al., Mol. Cell Biol., 5:410-413 (1985); and Kioosis, D. et al., EMBO, 6:355-361 (1987).

Higher levels of expression can be attained by removing the SV40 enhancer from the expression vectors formed from the pSV2-class vectors. Nearly all genomic immunoglobulin genes contain enhancer sequences. The murine kappa variable region gene is found on plasmid pGCEMK in conjunction with an approximately 300 base pair human kappa enhancer sequence, while the murine gamma variable region found on plasmid pNCEMG1 is found in conjunction with an approximately 180 base pair murine enhancer sequence. Skilled artisans will recognize that these enhancer sequences can be moved to many different sites on the expression vectors without changing the ability of transfected cells to produce these immunoglobulin chains. However, the removal of the SV40 enhancer from the expression vectors pGCEMK or pNCEMK leads to a marked increase in the levels of expression of chimeric CEM antibody from SP2/0 cells.

The CEM Kappa promoter, found on plasmid pGCEMK is also useful to increase the levels of expression of heterologous immunoglobulin chains. For example, CHA255 is a monoclonal antibody which specifically recognizes the EDTA chelate of indium. The lambda and gamma variable region-encoding genes from murine hybridoma CHA255 have been cloned. (See, for example, M.J. Johnson, Chimeric Antibodies Directed Against Metal Chelates, Attorney Docket No. H-7589, European Patent Appl. No.89302314.3, filed this even date, herein incorporated by reference.) Plasmid pGCHAK-2, which comprises the murine lambda CHA variable region and human constant region genes driven by the CEM kappa promoter on an SV40 enhancer-containing vector was constructed according to the teaching of Example 7. Plasmid pGCHAK-3, which comprises the murine lambda CHA variable region and human constant region genes driven by the CEM kappa promoter on an SV40 enhancerless vector was also constructed according to the teaching of Example 7. SP2/0 cells transfected by either of these plasmids, then subsequently transfected with a vector encoding the murine CHA gamma variable region joined to the human gamma constant region, demonstrated levels of expression which were much greater than cells transfected with vectors comprising the CHA lambda promoter. Indeed, those

6

cells which were transfected with SV40 enhancerless vectors comprising the CEM Kappa promoter driving the expression of the CHA chimeric genes displayed a ten-fold increase in the levels of kappa expression above the levels demonstrated by cells transfected with SV40 enhancer "plus" vectors comprising the CHA chimeric genes driven by the CHA lambda promotor. Subsequent transfection of these high kappa producing cells with vectors containing chimeric heavy chain genes gave a corresponding increase in whole antibody production over cells using the CHA promoter in the kappa chain vector. Even greater levels of expression can be attained by subcloning the highest expression cells, then growing these high expression subclones in well-defined, serum free media. Media such as HH2 (available from Hybritech Inc., Sand Diego, CA) or a wide variety of other commercially available media (Ventrex, Inc. or Gibco, Inc.) are all useful for growth of high expression clones. From this, it is apparent that the CEM kappa promoter has the specific ability to drive high levels of expression of both homologous (CEM) sequences and heterologous (CHA, or other) sequences. While the teaching of Example 7 demonstrates the presence of the CEM Kappa promoter on an approximately 2.2 kilo base ClaI/SspI restriction fragment, the CEM Kappa promoter sequence can easily be derived by those skilled in the art.

The eukaryotic host cells useful in the present invention are, preferably, hybridoma, myeloma, plasma cytoma or lymphoma cells. However, other eukaryotic host cells may be suitably utilized provided the mammalian host cells are capable of recognizing transcriptional and translational DNA sequences for expression of the chimeric genes; processing the leader peptide by cleavage of the leader sequence and secretion of the chimeric proteins; and providing post-translational modifications of the chimeric proteins, e.g., glycosylation.

Accordingly, the present invention provides eukaryotic host cells which are transformed by recombinant expression vectors comprising the chimeric gene constructs disclosed herein and which are capable of expressing the chimeric proteins of the invention. The term "transformation," as used herein, refers to the change in the genome of a host cell by introduction of DNA into the recipient cell. The transformed host cells of the invention, therefore, comprise at least one DNA construct comprising the chimeric light and heavy chain genes described herein, and transcriptional and translational sequences which are positioned in relation to the light and heavy chain-encoding DNA sequences to direct expression of these chimeric proteins.

The host cells used in the invention may be transformed in a variety of ways by standard transfection procedures well known in the art. Among the standard transfection procedures which may be used are electroporation techniques, protoplast fusion and calcium-phosphate precipitation techniques. Such techniques are generally described by Toneguzzo, F. et al., Mol. and Cell. Biol., 6:703-706 (1986); Chu, G., et al., Nucleic Acid Res., 15:1311-1325 (1987); Rice, D., et al., Proc. Natl. Acad. Sci. USA, 79:7862-7865 (1979) and; Oi, V., et al, Proc. Natl. Acad. Sci. USA, 80:825-829 (1983).

Preferably, the recombinant expression vectors comprising the chimeric constructs of the invention are transfected sequentially into host cells. For example, the expression vectors comprising the chimeric light chain DNA constructs are first transfected into the host cells and transformed host cells expressing the chimeric light chain polypeptides are selected by standard procedures known in the art. See, e.g. Engvall, E. and Perlmann P., Immunochemistry, 8:871-874 (1971). The expression vectors comprising the chimeric heavy chain DNA constructs are, thereafter, transfected into the selected host cells. However, it will be recognized that both the chimeric light and heavy chain expression vectors can be introduced simultaneously into the host cells. Alternatively, both the chimeric gene constructs can be combined on a single expression vector for trans fection into cells. Following transfection and selection, standard assays are performed for the detection of antibodies directed against CEA and identification of transformed cells expressing both the chimeric light and heavy chain genes defined by the invention.

The amino acid sequences of the chimeric polypeptides comprising the novel chimeric antibodies of the invention can be deduced from the DNA sequences disclosed herein. Accordingly, novel chimeric antibodies directed against CEA are provided comprising a variable light chain region having an amino acid sequence substantially the same as:

Asp - Ile - Val - Met - Thr - Gln - Ser - Gln - Lys
Phe - Met - Ser - Thr - Ser - Val - Gly - Asp - Arg
Val - Ser - Ile - Thr - Cys - Lys - Ala - Ser - Gln
Asn - Val - Arg - Thr - Ala - Val - Ala - Trp - Tyr
Gln - Gln - Lys - Pro - Gly - Gln - Ser - Pro - Lys
Ala - Leu - Ile - Tyr - Leu - Ala - Ser - Asn - Arg
Tyr - Thr - Gly - Val - Pro - Asp - Arg - Phe - Thr
Gly - Ser - Gly - Ser - Gly - Thr - Asp - Phe - Thr
Leu - Thr - Ile - Thr - Asn - Val - Gln - Ser - Glu
Asp - Leu - Ala - Asp - Tyr - Phe - Cys - Leu - Gln
His - Trp - Asn - Tyr - Pro - Leu - Thr - Phe - Gly
Ala - Gly - Thr - Lys - Leu - Glu - Leu - Lys - Arg.

Additionally, novel chimeric antibodies having specificity for CEA are provided which are comprised of a variable heavy chain region having an amino acid sequence substantially the same as:

Asp - Val - Gln - Leu - Val - Glu - Ser - Gly - Gly
Gly - Leu - Val - Gln - Pro - Gly - Gly - Ser - Arg
Lys - Leu - Ser - Cys - Ala - Ala - Ser - Gly - Phe

7

Thr - Phe - Ser - Asn - Phe - Gly - Met - His - Trp
Ile - Arg - Gln - Ala - Pro - Glu - Lys - Gly - Leu
Glu - Trp - Val - Ala - Tyr - Ile - Ser - Gly - Gly
Ser - Ser - Thr - Ile - Tyr - Tyr - Ala - Asp - Thr
Val - Lys - Gly - Arg - Phe - Thr - Ile - Ser - Arg
Asp - Asn - Pro - Arg - Asn - Thr - Leu - Phe - Leu
Gln - Met - Thr - Ser - Leu - Arg - Ser - Glu - Asp
Thr - Ala - Met - Phe - Tyr - Cys - Ala - Arg - Asp
Tyr - Tyr - Ala - Asn - Asn - Tyr - Trp - Tyr - Phe
Asp - Val - Trp - Gly - Ala - Gly - Thr - Thr - Val
Thr - Val - Ser - Ser - Ala.

The variable light and heavy chain regions, as described herein, may further comprise a eukaryotic leader peptide as described above. Preferably, the amino acid sequence of the leader peptide for the variable light chain region is substantially the same as:

Het - Glu - Phe - Gln - Thr - Gln - Val - Phe - Val
Phe - Val - Leu - Leu - Trp - Leu - Ser - Gly - Val
Asp - Gly.

The amino acid sequence of the leader peptide for the variable heavy chain region is, preferably, substantially the same as:

Met - Asp - Ser - Arg - Leu - Asn - Leu - Val - Phe - Leu
Val - Leu - Ile - Leu - Lys - Gly - Val - Gln - Cys.

Those skilled in the art will appreciate, however, that alternative amino acid sequences, as well as peptides derived from other secreted proteins, which function as leader peptides for the variable light and heavy chain regions defined herein may be suitably utilized in the invention.

Additionally, it is understood that minor modifications to the amino acid sequences of the chimeric polypeptides disclosed herein may result in variable regions which are substantially equivalent in the binding of CEA. These modifications are contemplated by the present invention provided the requisite specificity for CEA is retained.

The novel chimeric antibodies provided by the present invention are useful for both in vitro and in vivo application. For example, the chimeric antibodies of the invention may be utilized in in vitro immunoassays for the detection of CEA and monitoring of the tumor-associated antigen, e.g., during therapy. Moreover, because it is expected that immunogenicity will be substantially reduced, the chimeric monoclonal antibodies of the invention are highly desirable for in vivo diagnostic and therapeutic application. Accordingly, the chimeric monoclonal antibodies provided by the invention are of substantial utility for the in vivo imaging and treatment of tumors associated with colorectal and breast carcinomas as well as tumors of the gastrointestinal tract, lung, ovary, and pancreas. The chimeric antibodies of the invention may be used as unmodified antibodies or may be conjugated to suitable radionuclides, drugs or toxins for in vivo imaging or therapy. Additionally, antibody fragments retaining the essential finding function of the chimeric antibodies of the invention, or mixtures including the antibodies, may be utilized depending upon the particular clinical application of the invention.

The following examples and figures are offered for purposes of illustration of the present invention and are not intended to limit it in any way.

Figure 1 - restriction site and function maps of plasmids pMLCE-10 and pHKF-1.

For purposes of this disclosure the restriction site and function maps in these figures are not drawn exactly to scale.

Figure 2 - restriction site and function maps of plasmids pHKCE-10 and pGCEMK.

Figure 3 - restriction site and function maps of plasmids pMHCE-30 and pHG1Z.

Figure 4 - restriction site and function maps of plasmids pHGCEM-30 and pNCEMG1.

Figure 5 - a schematic of the construction of plasmid p19HANCH.

Figure 6 - restriction site and function maps of plasmid pGCHAK-3.

Figure 7 - restriction site and function maps of plasmids pUCVHInc-1A, pHG1-CHA and pNCHAG1.

Figure 8 - restriction site and function maps of plasmids pMLCH-1, pMLCH1dB and pGCHAK.

## Examples

Murine hybridoma cells, designated as CEM 231.6.7, were used in the examples to derive and clone genomic DNA for variable light and heavy chain variable regions. Murine hybridoma CEM 231.6.7 was deposited on January 7, 1988 with the American Type Culture Collection, Rockville, Maryland, under the accession number ATCC HB 9620. The cloned genomic DNA from murine hybridoma CEM 231.6.7, and human peripheral blood lymphocytes, were used for the construction of the chimeric genes.

Transfection of chimeric genes was accomplished by electroporation techniques, essentially as described by Toneguzzo, F., et al., Molecular and Cell. Biol., 6:703-706 (1986); and Chu, G. et al., Nucleic Acid Res., 15:1311-1325 (1987). The host cells SP2/O-Ag14 hybridoma cells were the recipients of the chimeric genes. The SP2/O-Ag14 hybridoma cells used are available from the American Type Culture Collection, Rockville, Maryland under the accession number ATCC CRL 1581.

## Example 1

### A. Isolation of DNA from CEM 231.6.7 Murine Hybridoma

Genomic DNA from CEM 231.6.7 was isolated by procedures essentially as described by Pellecer et al. , Cell, 41:133 (1978). Approximately 1 X $10^8$ cells were collected by centrifugation (10', 800 rpm IEC clinical centifuge). Cells were then washed 2x in Phosphate Buffered Saline (PBS). Cells were then resuspended in 4 ml of 10 mM Tris-HCl (pH 8.0) 2 mM EDTA, 40 mM NaCl (TEN), and lysed by the addition of 200 μl of 10% SDS and 42 μl of Protease K at 20 mg/ml (Sigma Chemicals, St. Louis, Missouri. The sample was incubated overnight at 37°C. DNA was extracted twice with an equal volume of phenol:chloroform:iso-amyl alcohol (25:24:1 ratio) and twice more with an equal volume of chloroform:iso-amyl alcohol (24:1 ratio) and dialyzed against 10 mM Tris-HCl (pH 8.0, 1 mM EDTA (TE Buffer) overnight. The DNA was then treated for 2 hours with RNASe A at 50 μg/ml (Sigma Chemicals, St. Louis, Missouri) followed by Protease K at 200 μg/ml for one hour at 37°C. DNA was extracted again as detailed above and dialyzed overnight. Following dialysis, DNA was ethanol precipitated by adding 1/10th volume of 2 M Na Acetate and 2 volumes of 95% ethanol. After 30 minutes at -20°C, the DNA was centrifuged at 8000 RPM for 30 minutes. The pellet was resuspended in TE buffer at a final concentration of 955 μg/ml.

### B. Construction of a Genomic Library for CEM231.6.7

Partial DNA digests of the CEM 231 genomic DNA were done by taking 10 μg of DNA in 11 μl TE buffer, 20 μg of $H_2O$, 15 μl of 10x Core Restriction Digest Buffer (500 mM pH8.0, 100 mM $MgCl_2$, 500 mM NaCl) and then aliquoted into tubes. Restriction enzyme Mbo I was added to each tube in units increasing from .0038 to .5 units/μl and placed at 37°C for one hour. Aliquots of the samples were then run on a 0.7% TBE (89 mM Tris 89 mM Borate 2 mM EDTA) agarose gel and electrophoresed overnight at 40 volts. From the photograph of this gel it was determined which digestion fractions contained DNA in the correct molecular weight range (12-24 kb). Two hundred μg of genomic DNA was then digested using the Mbo I units defined by the experiment described above (scaled up 20x) with an hour incubation at 37°C. This DNA was used to construct the EMBL-3 phage library using EMBL-3 phage DNA, commercially available from Stratagene, Inc. (La Jolla, CA), as described in the Stratagene protocol. The Stratagene protocol follows the techniques described in several laboratory manuals (e.g., Basic Methods in Molecular Biology, edited by L.G. Davis, M.D. Dibner and J.F. Battey, Elsevier, New York (1986). Following isolation on sucrose gradients, the large molecular weight CEM 231.6.7 DNA (12-24 kb) was ligated into the EMBL-3 phage arms using 100 ng of DNA, 100 ng of pre-isolated EMBL-3 phage arms, .5 μl of 10 x Ligase Buffer (500 mM Tris-HCl pH8; 70 mM $MgCl_2$; 10 mM dithiothreitol (dtt) and T4 DNA ligase (2 units) at 4°C, overnight. Packaging was done using Stratagene's commercially available Gigapack-Gold In Vitro packaging system in accordance with their product protocols and using the host E. coli strain P2.392 supplied by Stratagene, Inc. 500 μl of Gigapack Gold packaging mix and 5 μl of the ligated phage were incubated at 22°C for 2 hours. 0.5 ml of phage dilution buffer (per liter 5.8 g NaCl, 2 g $MgSO_4$-$6H_2O$, 50 ml 1M Tris-HCl pH 7.5, 5 ml 2% gelatin) was added. Phage were then titered using standard protocols as described in Molecular Cloning, edited by T. Maniatis, E.F. Fritsch and J. Sambrook, Cold Spring Harbor Laboratories, Cold Spring Harbor, New York (1982). Following titration, the phage library was plated at a density of 20,000 plaques/100 mM plate using P2.392 cells and incubated at 37°C overnight.

### C. Identification and Isolation of Recombinant Phage ØMLCE-10 Including Murine CEM 231.6.7 v Kappa Gene

For the CEM 231.6.7 light (kappa) chain 4.8 x $10^5$ recombinant phage in E. coli P2.392, which is commercially available from Stratagene, Inc. (La Jolla, CA) were screened according to the protocol in Molecular Cloning, supra. This was done using murine kappa and murine $J_L$ probes derived from plasmids obtained from Dr. Marc Shulman, University of Toronto, Toronto, Canada or synthetic probes whose sequence was derived from the General Bank Data Base (NIH Accession #J00545). The kappa oligonucleotide probe used was comprised of the sequence 5'-AGA-TGG-ATA-CAG-TTG-GTG-CAG-CAT-CAG-CCC-3' and was synthesized by Molecular Biosystems, Inc. (San Diego, CA).

Duplicate filter lifts were made and phage hybridizing to both probes were analyzed by double southern blots as described in Molecular Cloning, supra, probed with the murine kappa and $J_L$ probes which were radiolabeled with $^{32}P$. Hybridizations were done as described in Molecular Cloning, supra. A single CEM 231.6.7 phage clone was selected on the basis of its hybridizing to both probes, indicating a light chain variable region gene rearrangement to a position directly contiguous to the C kappa gene region. This recombinant phage was designated ØMLCE-10.

### D. Construction of Recombinant Plasmid pMLCE-10 Including CEM 231.6.7 Murine v Kappa Gene

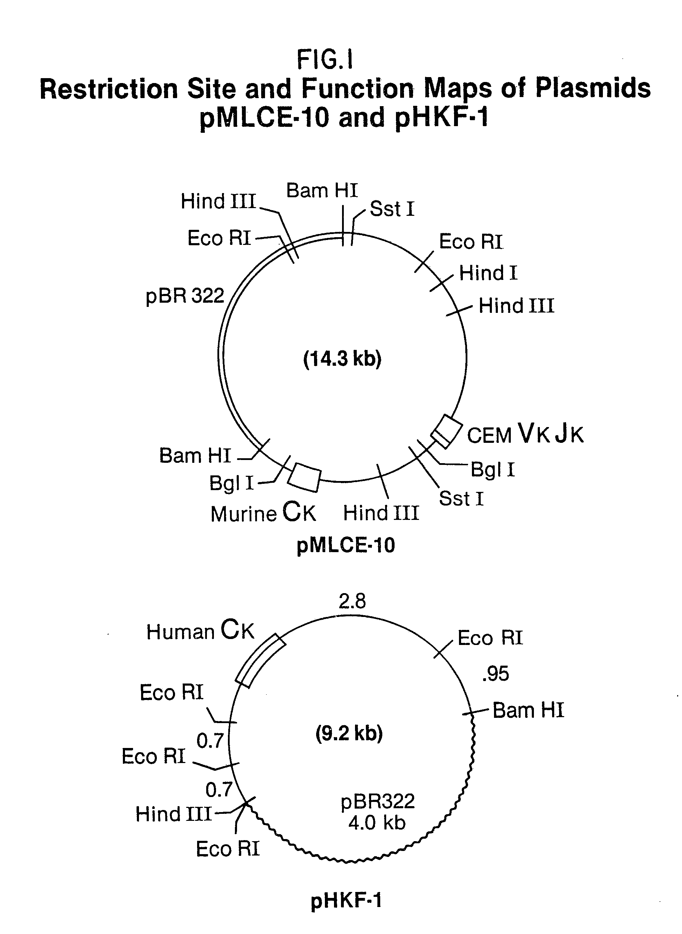

ØMLCE-10 DNA was isolated and 20 μg were digested with BamHI (1 unit/μg) using React Buffer #3 from Gibco-BRL (Gaithersburg, Maryland) in a total of 220 μl. A 10 kb BamHI fragment was isolated by electrophoresis of the BamHI digested DNA in a .75% TBE agarose gel containing .5 μg/ml of ethidium bromide at 40 V overnight. Following visualization on a UV transparent light box the 10 kb fragment was electrophoresed onto DEAE 81 (Schleicher and Schuell, Keene, New Hampshire) paper followed by elution in 1 M NaCl and ethanol precipitation. The eluted fragment was then resuspended in 6 μl of TE buffer. The fragment was ligated to 50 ng of BamHI digested pBR322, available from the American Type Culture Collection (ATCC Designation 31344) by using 1 μl (50 ng) of pBR322 DNA, 6 μl (300 ng) of BamHI 10 kb recombinant phage DNA ligase as described in Example 1B. E. coli HB101 competent cells, available from Gibco-BRL (Gaithersburg, Maryland), were transformed using standard procedures. HB101 cells were first thawed on ice; then 10 μl of the ligation reaction were mixed with 200 μl of cells and incubated on ice for 30 minutes. Following this, cells were heat shocked for 45 seconds at 42°C, then returned to ice for 2 minutes. One ml of LB broth (per liter 10 g NaCl, 5 g yeast extrct 10 g tryptone) was added and the cells incubated in a New Brunswick air shaker for one hour at 225 rpm, 37°C. Two hundred μl were then plated on LB-agarose with ampicillin (50 μg/ml) plated and incubated overnight at 37°C. Ampicillin resistant colonies were screened using colony screening methods as detailed in Molecular Cloning, supra, and Grunstein, M. and Hogness, D., Proc. Nat. Acad. Sci., USA, 72:3961 (1975). Again the $J_L$ and Kappa murine probes were used to identify the recombinant pBR322 plasmid, designated as pMLCE-10 and was deposited under the Budapest Treaty with the American Type Culture Collection on March 1, 1988 (ATCC Deposit #67639). The restriction site and function map of plasmid pMLCE-10 is presented in Figure 1 of the accompanying drawings.

### E. Isolation of Human DNA
Whole blood was obtained from individuals homozygous for the human gamma haplotypes fbn and azg in 30 ml samples. Buffy coat cells were harvested from the blood samples by centrifugation at 2500 rpm in a Sorvall GLC-2B at 22°C. The buffy coat cells were removed with a pasteur pipet and washed once with PBS. The cell pellets were resuspended in 500 μl of 10 mM Tris-HCl (pH 8.0) 40 mM NaCl and lysed by the addition of 30 μl 10% SDS and 6 μl 20 mg/ml Protease K (Sigma Chemicals, St. Louis, Missouri). The sample was incubated 15 hours at 37°C. The DNA was extracted twice with an equal volume of phenol:chloroform:isoamyl alcohol (25:24:1), twice more with chloroform:isoamyl alcohol (24:1) and dialyzed against 10 mM Tris-HCl (pH 8.0) 1 mM EDTA. The DNA was then treated for two hours with 50 μg/ml RNAse A (Sigma Chemicals) followed by redigestion with 200 μg/ml Protease K for 1 hour. The DNA was extracted and dialyzed as described above and the concentration determined by $OD_{260}$ to range from 100-200 mg/ml.

### F. Construction of a Human Genomic Phage Library
Human DNA of the fbn haplotype (210 μg as isolated in Example 1E) was partially digested with Mbol, DNA fragments in the molecular weight range 12-24 kb isolated, and cloned into EMBL-3 phage as described in Example 1B.

### G. Isolation of Recombinant Plasmid pHKF-1
In order to isolate the human kappa constant region gene, the EMBL-3 library, as described in Example 1B, was screened with a human kappa probe supplied by Dr. P. Hieter, Johns Hopkins University, Baltimore, Maryland, the sequence of which is available from the NIH Data Base, accession number J00241. A total of 5 x $10^5$ recombinant phage were screened as described in Example 1C. A single clone was isolated and designated ØHKF-1. Fifteen μl of ØHKF-1 DNA were digested with BamHI and HindIII in React Buffer #3 (Gibco-BRL, Gaithersburg, Maryland). A 5.2 kb fragment was isolated on DEAE 81 paper and ligated into the cloning vector pBR322 as described in Example 1D. Ampicillin resistant recombinant colonies were again identified using the human kappa probe; a single clone was isolated and designated pHKF-1. pHKF-1 was deposited under the Budapest Treaty with the American Type Culture Collection on March 1, 1988 (ATCC Deposit #67637). The restriction site and function map of plasmid pHKF-1 is presented in Figure 1 of the accompanying drawings.

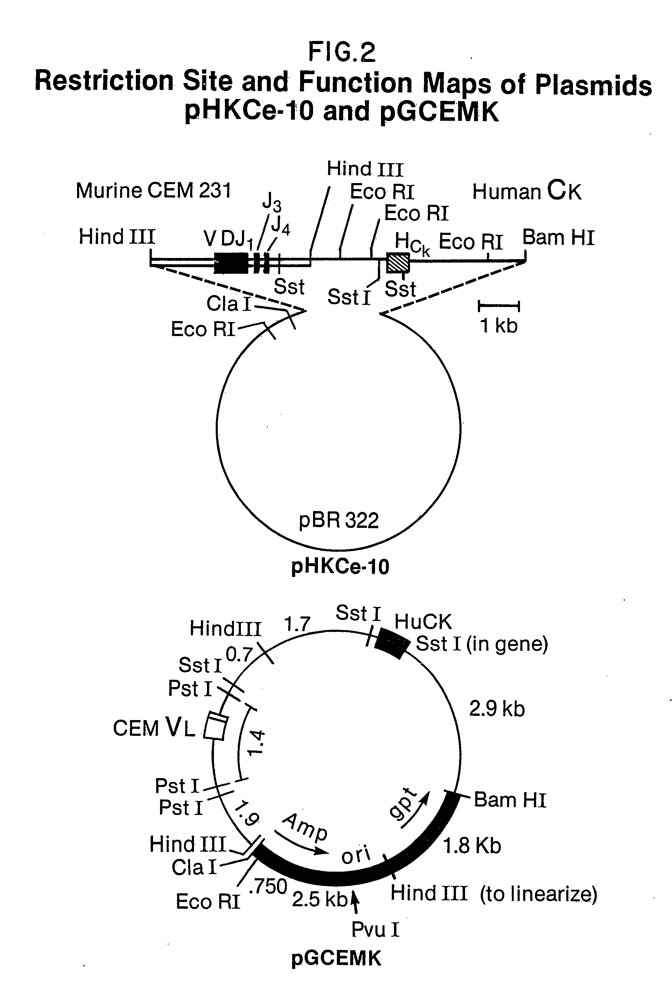

### H. Construction of Plasmid pHKCE-10 Including CEM 231.6.7 $V_L$ Kappa Gene and Human C Kappa Gene
A 3.8 kb HindIII fragment from pMLCE-10 containing the entire CEM 231.6.7 variable kappa region was further subcloned into the HindIII site of plasmid pHKF-1, described in Example 1E, using the methods described in Example 1D to form a chimeric plasmid having the murine CEM 231.6.7 variable light region fused to a human constant kappa region gene. One μg of pMLCE-10 DNA was digested with HindIII at 1U/μg using React Buffer #2 (Gibco-BRL, Gaithersburg, Maryland) and of pHKF1 was digested in a similar manner. The pMLCE-10 digested DNA was electrophoresed as above and the 3.8 kb HindIII fragment was isolated onto DEAE 81 paper and eluted in 5 μl of TE. One μg (2 μl) of HindIII digested pMLCE-10 were ligated to 600 ng of HindIII digested pHKF-1 in the presence of 10x Ligation Buffer, 10 mM ATP and 2 units of T4 DNA ligase of total volume of 10 μl. Ligation was done at 12°C overnight and again HB101 competent cells from BRL were used in the plasmid transformation, described in Example 4. The plasmid designated as pHKCE-10 was identified by restriction mapping of the plasmid DNA. The restriction site and function map of plasmid pHKCE-10 is presented in Figure 2 of the accompanying drawings.

### I. Construction of Plasmid pGCEMK Including Chimeric Light Chain Immunoglobulin Genes
The eukaryotic expression vector containing the murine $V_L$ region fused to the human kappa gene was constructed using the vector pSV2gpt, available from the American type Culture Collection, (ATCC Designation #37145). One μg of pSV2gpt DNA was digested with the restriction enzyme Eco R1 using 1 unit/µg of DNA in Reaction Buffer #3 (Gibco-BRL, Gaithersburg, Maryland). The Eco R1 ends were then made blunt by adding 10 µl of 5 mM each of the 4 deoxyribonucleotides dTTP, dGTP, dCTP and dATP, 2 units of Klenow enzyme and a 10x buffer (.5M Tris HCl pH 7.5; .1 M MgCl₂; 10 mM diothiothreitol) in a total 50 µl as described in Molecular Cloning, supra. The reaction went 30 minutes at room temperature and was followed by a ligation reaction in which phosphorylated Cla I linkers (2 µg) (New England BioLabs, Beverly, Massachusetts) were ligated to the 500 ng of Eco R1 blunted ended pSV₂gpt in order to create a new Cla I site for our future chimeric vector. The Cla I linkers sequence was d(pCATCCGATG). Ligation reactions were carried out as described under Example 1B. Following ligation, excess linkers were removed by electrophoresis of the DNA and isolation of the linear pSV₂gpt-Cla fragment onto DEAE 81 paper as in Example 1D. The isolated DNA was self-ligated using reagents described in Example 1B. HB101 competent cells were transformed as in Example 1D and the ampicillin resistant colonies analyzed by restriction enzyme digestion.

The resulting vector, pSV2gpt-Cla, was then digested with Cla I and BamHI restriction enzymes (1 unit/µg of DNA). The chimeric vector pHKCE-10 was also digested with these two enzymes. The 4.5 kb pSV2gpt Cla-Bam fragment and the 9 kb Cla-Bam pHKCE-10 fragment were isolated on DEAE 81 paper as described in Example 1D. A standard ligation reaction as described above was done using 375 ng of the 9 kb fragment insert DNA and 200 ng of the 4.5 kb vector DNA. Following transformation of HB101, a recombinant plasmid, designated as pGCEMK, was identified by restriction mapping of the plasmid DNA. This plasmid, which is the chimeric expression vector used to produce the CEM chimeric light chain polypeptide, is shown with restriction sites in Figure 2.

### J. Isolation of the Recombinant Phage ØMHCE-30 Including CEM 231.6.7 V_h Gene

In order to identify the CEM 231.6.7 gamma chain, the EMBL-3 library described in Example 1B was screened with two murine heavy chain probes, one representing the J_h 3-4 regions was obtained from Dr. Phil Tucker, University of Texas, Dallas, Texas, and one representing sequences in the murine gamma-1 gene. This latter probe was comprised of the sequences 5'-CTG-TAC-ATA-TGC-AAG-GCT-TAC-AAC-CEC-AAT-3' as determined from the General Bank Data Base (NIH Accession #J00453) and was synthesized by Molecular Biosystems, Inc. (San Diego, CA). A total of 4.8 x 10⁵ recombinant phage plaques were screened in duplicate using these two probes in order to identify the clone containing both the J_h region and the gamma region. Again, as with the kappa clone, a phage containing sequences for these two regions indicates that that DNA has been rearranged, thus identifying the expressed immunoglobulin gene in the cell line CEM 231.6.7. A single CEM 231.6.7 clone, was selected on the basis of its being rearranged and was designated ØMHCE-30.

### K. Construction of pMHCE-30 Including CEM 231.6.7 V_h Gene

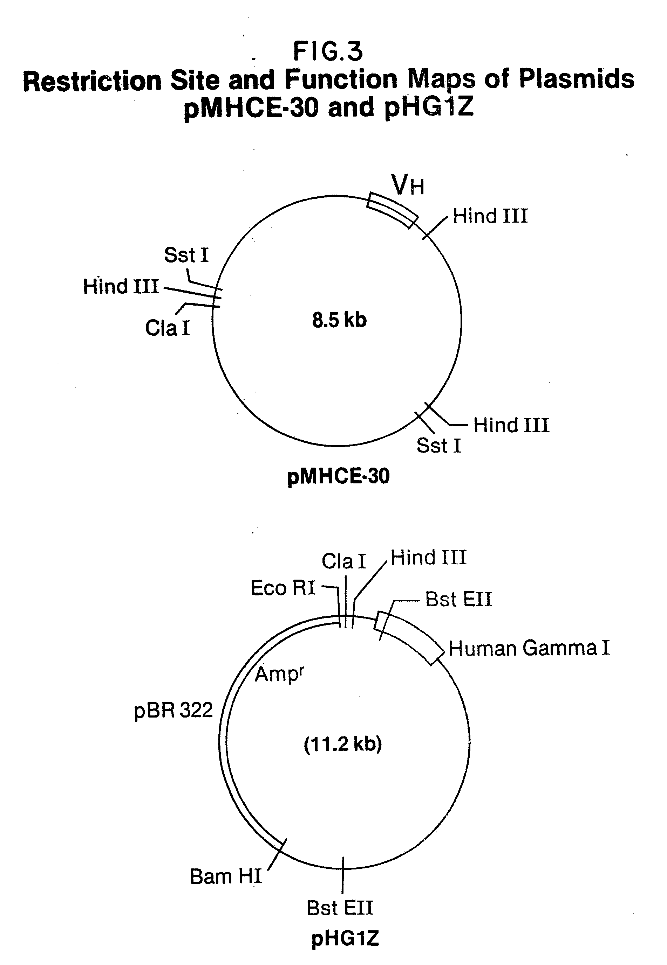

By restriction mapping, a 5.6 kb Sst I fragment from ØMHCE-30 was identified which contained the heavy chain variable region sequences as well as the major intron containing the murine heavy chain enhancer. To subclone this fragment into a plasmid, 10 µg of the page DNA was digested with the restriction enzyme Sst I in Reaction Buffer #2 (BRL-Gibco, Gaithersburg, Maryland) and the 5.6 kb fragment was isolated following electrophoresis by the DEAE 81 method described in Example 1D. The Bluescript vector M13-SK+, commercially available from Stratagene, Inc. (La Jolla, CA) was also digested with Sst I. The vector and isolated 5.6 kb insert DNA was ligated at a ratio of 1:10 in a total of 10 µl as detailed in Example 1B. Following transformation of HB101 competent cells, the correct recombinant was identified by restriction digest mapping, and designated as pMHCE-30. pMHCE-30 was deposited under the Budapest Treaty with the American Type Culture Collection on March 1, 1988 (ATCC Deposit #67640). The restriction site and function map of plasmid pMHCE-30 is presented in Figure 3 of the accompanying drawings.

### L. Isolation of Human DNA

Human DNA was isolated as described and detailed in Example 1E above.

### M. Construction of a Human Plasmid Library

Human DNA of the azg haplotype was digested as 10 µg aliquots using 30 units each of the restriction endonucleases BamHI and HindIII in Reaction Buffer #3 (Gibco-BRL, Gaithersburg, Maryland) (50 mM Tris-HCl pH 8.0, 10 mM MgCl₂, 100 mM NaCl) in a total volume of 200 µl. The digested fragments were concentrated to 20 µl by ethanol precipitation and separated on a 0.6% low gelling temperature agarose (FMC) gel run for 15 hours at 50 mAmps. DNA fragments in the size range 6-7 kb were excised from the gel. The cloning vector pUC 18 (described by Yanisch-Perron C., et al., Gene, 33:103 (1985)) was also digested with BamHI and HindIII as described above. The human DNA fragments (150 ng) were ligated into the pUC 18 vector (50 ng) (New England BioLabs, Beverly, Massachusetts) in a total reaction volume of 400 µl containing 30 mM Tris-HCl (pH7.6), 10 mM MgCl₂, 5 mM dithiothreitol, 1 mM ATP, and 1 µl T4 DNA ligase (2 units, for 72 hours at 15°C. Half (100 ng) of the ligated DNA sample was used to transform 500 µl of freshly prepared competent E. coli M15 cells and the resulting transformants were plated onto X-gal, IPTG, AMP plates (4 µg/ml X-gal, 2 µg/ml IPTG, 100 µg/ml Ampicillin).

11

### N. Isolation of Recombinant Plasmid pHG1Z

Ampicillin resistant pUC18 colonies containing recombinant human DNA were screened by the method described in Example 1 using a human Gamma 2 probe supplied by T.Honjo, University of Osaka, Japan and described by Takahashi, N. et al., Cell., 29:671-679 (1982). A clone containing a 7.5 kb insert corresponding to the human Gamma 1 gene was identified and designated HyHG1. This same 7.5 kb HindIII-BamHI fragment containing the human gamma constant region gene was subsequently re-cloned into pBR322 using the same methodology as described in Example 1G. The pBR322 vector containing the human gamma 1 gene was designated pHG1Z and was deposited under the Budapest Treaty with the American type Culture Collection on March 1, 1988 (ATCC Deposit #67638). The restriction site and function map of plasmid pHG1Z is presented in Figure 3 of the accompanying drawings.

### O. Construction of pHGCEM-30 Including CEM 231.6.7 $V_h$ Gene and Human Gamma-1 Gene

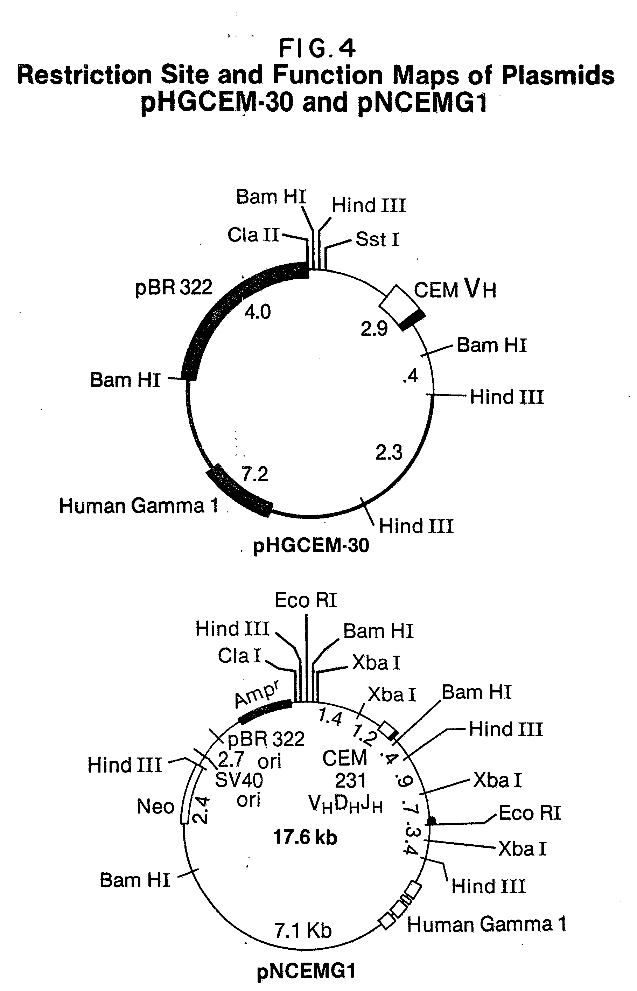

The murine variable heavy chain region was fused to the human gamma-1 gene in the following manner. Ten μg of pMHCE-30 was digested with Cla I (1 unit/μg) and then partially digested with HindIII to give a 5.3 kb fragment containing $V_h$ and the major intron. Partial digests were performed by using only .1 unit/μg of DNA and a digestion time of 1 hour at 37°C. One μg of the plasmid pHGZ-1, described in Example 1N containing the human gamma 1 gene was also digested with Cla I and HindIII. The 5.3 kb fragment from pMHCE-30 was isolated from a TBE gel using the DEAE 81 protocol as described in Example 1D. This fragment was ligated into the Cla-Hind site of pHGZ-1 by using 500 ng of the insert and 200 ng of the vector DNA in a ligation mixture of 10 μl total volume. Ligation reactions were carried out as detailed in Example 1B. The recombinant plasmids resulting from transformation of HB101 were analyzed by restriction digest mapping in order to identify a plasmid containing the murine $V_h$ region fused to a human gamma 1 gene, and was designated pHGCEM-30. The restriction site and function map of plasmid pHGCEM-30 is presented in Figure 4 of the accompanying drawings.

### P. Construction of pNCEMG1 Including Chimeric Heavy Chain Immunoglobulin Genes

The chimeric Ig gene was inserted into the eukaryotic expression vector essentially as detailed in Example 1H. The vector used was pSV2neo, available from The American Type Culture Collection (ATCC Designation #37149). A Cla I site was added to this vector exactly as described for the pSV2gpt vector. Following this, 1 μg of pSV2neo-Cla DNA was digested with the enzymes Cla I and Bam HI using 1 unit/μg of DNA. The plasmid pHGCEM-30 was digested with Cla I and then partially digested with Bam HI (.1 unit/μg) in order to obtain a fragment of 12.7 kb which contained the chimeric $V_h$ and gamma 1 regions. This fragment was isolated on DEAE 81 paper and eluted in 10 μl of TE buffer. The ligation was done using 50 ng of vector DNA, 400 ng of the 12.7 kb insert DNA, 10x ligation buffer, 10 mM ATP and T4 DNA ligase, as in Example 1B at 12°C overnight. HB101 cells were transformed and the correct recombinant plasmid constituting the chimeric expression vector pNCEMG1 was identified. pNCEMG1, which constitutes the recombinant expression vector used to express chimeric heavy chain immunoglobulin genes and is shown with restriction sites, in Figure 4.

### Q. Construction of plasmid pNCEMK

Plasmid pNCEMK was constructed in essentially the same manner as plasmid pNCEMG1, except that the approximately 9.0 kb ClaI/BamHI fragment of plasmid pHKCE-10 (isolated in Example 1I) was ligated into the ClaI/BamHI-digested plasmid pSV2neo-Cla.

### R. Construction of plasmid pGCEMG1

Plasmid pGCEMG1 was constructed in essentially the same manner as plasmid pGCEMK (see Example 1I), except that the approximately 12.7 kb ClaI/BamHI (partial) restriction fragment of plasmid pHGCE-30 (see Example 5C) was ligated into the ClaI/BamHI-digested plasmid pSV2gpt-Cla.

## Example 2

### DNA Sequencing of Cloned Genes

Sequencing of the cloned CEM variable light and heavy chain genes was accomplished by standard procedures for both double and single stranded templates using the protocols provided by the sequencing kit Sequenase, commercially available from U.S. Biochemicals (Cleveland, Ohio), and the Bluescript/DNA Sequencing System, commercially available from Stratagene, Inc. (La Jolla, CA). From the DNA sequences obtained for the cloned CEM variable light and heavy region genes, the amino acid sequences of the polypeptides encoded for were deduced by a computer software program, MAPSEQ, commercially available from DNAStar, (Madison, Wisconsin).

## Example 3

## A. Transfection of Chimeric Light Chain Gene with the Chimeric Construct pGCEMK

The light chain immunoglobulin plasmid used for transfection was pGCEMK, as described in the Example 1 above. The pGCEMK plasmid, containing the chimeric variable light ($V_K$) CEM gene fused to the human kappa gene, was first transfected into SP2/0 hybridoma cells by the electroporation techniques referenced above. The SP2/0-Ag14 cells were grown in media containing 5% FCS and maintained in a log phase of growth for the three days preceeding electroporation. Twenty μg of the plasmid vector pGCEMK was linearized using the restriction enzyme Pvul (1 u/μg) and the Reaction Buffer #7 (Gibco-BRL, Gaithersburg, Maryland). At the time of transfection the SP2/0 cells were collected by centrifugation in an IEC clinical centrifuge - 800 rpm 10' room temperature. Cells were then washed 3x in Hanks Buffered Saline Solution (Gibco Laboratories, Grand Island, New York) with 6 mM Dextrose and resuspended at a final concentration of $3.0 \times 10^7$ cells/ml. 0.3 mls of cells were aliquoted into cuvettes at a density of $1 \times 10^7$/.3 ml and the linearized DNA was added. The mixture was maintained on ice 10 minutes. Electroporation was done using the .8 mm gap electrode (P/N 472) and the BTX 100 Transfector (BTX, Inc. San Diego, CA.). Conditions were 3 pulses, 100 μ seconds each at 300 volts. The electroporated cells were then resuspended in medium at a density of $2 \times 10^5$/ml (in T75 flasks) for 72 hours. ($37^\circ$ C 5% $CO_2$). Cells were then plated in the appropriate antibiotic at a density of $5 \times 10^4$/ml in 24 well plates; SP2/0 cells containing pGCEMK were plated in HMAX 1.0 Media (50 ng/ml Hypoxanthine, 250 ng/ml Mycophenolic Acid and 50 μg/ml Xanthine), available from Sigma, St. Louis, Missouri, at 1 μg/ml. Two hundred μl of supernatant was collected from each well which contained HMAX resistant colonies. This supernatant was then assayed for the presence of a human kappa constant region gene which would indicate expression of the chimeric immunoglobulin genes of pGCEMK.

## B. Identification of SP2/0 Cells Secreting Chimeric CEM 231.6.7

Transfected SP2/0 cells expressing the chimeric CEM-human kappa genes were identified by a standard enzyme-linked immunosorbent assay (ELISA), as described by Engvall, E. and Perlmann, P., Immunochemistry, 8:871-874 (1971), for human kappa.

The purpose of this assay was to identify those cells secreting the chimeric kappa chain polypeptide coded for by the pGCEMK plasmid vector which was constructed from murine variable regions isolated from the murine hybridoma CEM 231.6.7 and fused to the human gamma 1 gene. A 5 μg/ml solution of goat anti-human kappa chain (Tago #4106) in 10 mM sodium phosphate pH 7-8 was prepared. Each well of a 96 well plate was coated with 50 μl of this solution. The plates were then incubated overnight at $37^\circ$ C. Plates were then rinsed thoroughly in $H_2O$ and PBS + 0.1% Tween (w/v). Fifty μl of the supernatant fractions were added to each well, and incubated for 2 hours at room temperature. Plates were again rinsed as detailed above. A goat anti-human kappa chain alkaline phosphatase conjugate (Tago #2496) was diluted 1:1000 in the same medium as the supernatant material. 100 μl were added per well and allowed to incubate for 1 hour at room temperature. Plates were rinsed as above. The alkaline phosphatase substrate was prepared as per package instruction, one tablet per 3 ml of distilled $H_2O$ and 150 μl of this substrate was added to each well and allowed to incubate 30 minutes at $37^\circ$ C. The reaction was quenched with 50 μl of 300 mM EDTA and then the absorbance was read at 405 nM. Those supernatants showing the highest levels of kappa expression were identified and the cells from the corresponding wells were pooled and expanded for introduction of the chimeric construct pNCEMG1.

## C. Transfection of Chimeric Kappa Producing Cells with the Heavy Chain Chimeric Construct pNCEMG1

The heavy chain immunoglobulin plasmids used for transfection into SP2/0 cells was pNCEMG1, derived from constructs as detailed in Example 1. The populations of cells expressing the chimeric CEM-human kappa genes which were pooled were next electroporated with the plasmid constructs containing the chimeric CEM heavy chain genes. As for the kappa gene electroporation the SP2/0 chimeric dappa producing cells (SP2/0-K) were maintained at log phase of growth for the three days preceeding the electroporation. Twenty micrograms of the plasmid DNA pNCEMG1 was linearized with the enzyme Pvu I in React buffer #6 (Gibco-BRL, Gaithersburg, Maryland). Cells were collected, washed and resuspended at a density of $3 \times 10^7$ cells/ml as detailed in Example 2A. The DNA was added and the mixture held on ice for 10 minutes preceeding the electroporation. Conditions used were 1 pulse at 5 m seconds, 250 volts. Cells were plated at $2.5 \times 10^5$/ml in mammalian tissue culture media, such as HH2 (or any other media such as DMEM or RPMI) plus 5% FCS plus HMAX 1.0 for 72 hours at $37^\circ$ C, 5% $CO_2$. Following, these cells were plated at $5 \times 10^4$/ml in 24 well plates in medium containing HMAX 1.0 and G418 antibiotic (Geneticin, Gibco-BRL, Gaithersburg, Maryland) at an active concentration of 500 μg/ml. Selection was maintained for 14 days at which time those wells with HMAX/G418 resistant colonies were identified for further analysis.

## Example 4

## Identification and Analysis of Chimeric Antibody SP2/0 Cells Secreting CEM-Chimeric Antibody

## A. Antigen Binding

Screening assays were performed to identify transfected SP2/0 cells which expressed both the chimeric CEM light and heavy chain immunoglobulin genes, producing antibody which binds CEA. The assay procedures used for the detection of antibodies directed against CEA were standard solid phase radioimmunoassays, essentially as described by Wang, R., et al., Immunol. Methods, 18:157-164 (1977).

The following reagents were added to wells of a microtiter plate and incubated overnight at room temperature with mixing: 25 μl cell culture supernatant, 50 μl $^{125}$I-CEA (affinity purified), 20 μl of Sepharose bound goat anti-human IgG and 25 μl cell culture media. Immune complexes bound to the Sepharose-anti-human IgG were collected onto paper filters. Filters were counted in a gamma counter. The radioimmunoassays resulted in the identification of a chimeric anti-CEA specific antibody, which was designated as XCEM 449.

## B. Antibody Affinity

Affinity assays were performed to determine the Ka of chimeric antibody XCEM 449 for CEA. The affinity of chimeric antibody XCEM 449 for CEA was determined by standard radioimmunoassay procedures, as described in Example 4A, and Scatchard analysis as described by Scatchard, G., Ann. New York Acad. Sci., 51:660 (1949).

Antigen binding assays were done as described in Example 4A. To generate the inhibition curve, 25 μl volumes of CEA were added to each reaction, substituting for the 25 μl of cell culture media. The mass of competitor added ranged from 1-100 ng. For the Scatchard analysis, a plot of bound/free vs bound antigen permitted calculation of the affinity constant as defined by the megative of the slope of the line. Affinities of the chimeric antibodies were at least comparable to those of the murine antibody counterparts from which the chimeric antibodies of the invention were derived.

It will be apparent to those skilled in the art that modifications and changes to the invention will be possible without departing from the spirit and scope of the invention. It is intended that the following claims be interpreted to embrace all such modifications and changes.

## Example 5

### Construction of an SV40 Enhancerless Cloning System

#### A. Construction of Plasmid pSV2gpt(E-)

About 20 μl (10 μg) of plasmid pSV2gpt-Cla (constructed in Example 1I) were mixed with 21 μl water, 5 μl Gibco Reaction Buffer #6, 2 μl restriction enzyme PvuII and 2 μl restriction enzyme SphII, then incubated for 2 hours at 37°C. The reaction mixture was electrophoresed through a 0.5% TBE gel and the about 4.9 kb PvuII/SphII-digested vector fragment was purified from DEAE 81 paper in substantial accordance with the teaching of Example 1D. To fill in the 3' protruding ends of these restriction sites, about 20 μl of the PvuII/SphII-digested pSV2gpt-Cla vector were mixed with 3 μl 10x T4 polymerase buffer (700 mM Tris, pH 7.4; 100 mM MgCl₂; 50 mM DTT), 3 μl of dNTP mix (0.5 mM each of dATP, dTTP, dCTP and dGTP, pH 7.0), 3 μl of water and 1 μl (5U) of T4 DNA polymerase (BRL). The mixture was incubated at 37°C for 15 minutes, then heated to 70°C for 10 minutes. After a phenol extraction and ethanol precipitation, the DNA was resuspended in 5 μl of TE buffer. About 1 μl (about 500 ng) of this DNA fragment was mixed with 10 μl of water, 5 μl of 5x ligation buffer, 2 μl of 100 μMATP and 2 μl of T4 ligase. After an incubation of 2 hours at room temperature, the ligation reaction was transformed into E. coli HB101 cells in substantial accordance with the teaching of Example 1P. Plasmid DNA was isolated from the trans formants and those plasmids which displayed the proper ~0.2 kb deletion of the SV40 enhancer region were designated plasmid pSV2gpt(E-).

#### B. Construction of Plasmid pSV2neo(E-)

An SV40 enhancerless pSV2neo was also constructed. About 20 μl (10 μg) of plasmid pSV2neo-Cla (constructed in Example 1P) was digested with restriction enzymes BamHI and HindIII in a reaction mixture using Gibco Reaction Buffer #3, substantially as described above. The approximately 2.3 kb HindIII/BamHI neomycin resistance-conferring gene containing fragment was isolated and purified from DEAE 81 paper following electroelution. In a similar manner, plasmid pSV2gpt(E-) was also digested with restriction enzymes BamHI and HindIII, and the large vector fragment was purified after electrophoresis. The about 2.3 kb HindIII/BamHI neo containing restriction fragment of plasmid pSV2neo-Cla was then ligated into the HindIII/BamHI-digested vector fragment of plasmid pSV2gpt(E-) in substantial accordance with the teaching of the previous paragraph. After transformation into E. coli HB101 and isolation of plasmid DNA, those plasmids in which the pSV2gpt(E-) vector backbone was ligated with the about 2.3 kb BamHI/HindIII neo fragment of plasmid pSV2neo-Cla were designated plasmid pSV2neo(E-).

#### C. Construction of Plasmids pGCEMK(E-) and pGCEMG(E-)

About 10 μg (100 μl) of plasmid pSV2gpt(E-)DNA were mixed with 4 μl of water, 12 μl of Gibco Reaction Buffer #1, 2 μl of restriction enzyme ClaI and 2 μl restriction enzyme BamHI, then incubated overnight at 37°C.

14

After electrophoresis onto DEAE 81 paper, the large vector fragment was purified and resuspended in 10 μl TE. About 20 μl (5 μg) of plasmid pHKCE-10 (constructed in Example 1H) were mixed with 23 μl water, 5 μl of Gibco Reaction Buffer #1 and 2 μl restriction enzyme ClaI. After 5 hours at 37°C, the reaction was extracted with phenol/chloroform and ethanol precipitated. This digested DNA was then resuspended in 25 μl of water and mixed with 3 μl of Gibco Reaction Buffer #3 and 2 μl Restriction enzyme BamHI. Following a 2 hour incubation at 37°C, the digested DNA was electrophoresed and the approximately 9.0 kb murine variable, human construct kappa-encoding ClaI/BamHI restriction fragment was purified from DEAE 81 paper. This approximately 9.0 kb fragment was then ligated into the ClaI/BamHI-digested plasmid pSV2gpt(E-) and transformed into E. coli cells as previously described. After plasmid isolation, those plasmids which comprise the correct restriction maps were designated plasmid pGCEMK(E-).

In a similar fashion, about 40 μl (5 μg) of plasmid pHGCE-30 (constructed in Example 10) were mixed with 3 μl of water, 5 μl of Gibco Reaction Buffer #1 and 2 μl restriction enzyme ClaI. After 5 hours at 37°C, the reaction was extracted with phenol/chloroform and ethanol precipitated. This digested DNA was resuspended in 26 μl of water, 3 μl of Gibco Reaction Buffer #3 and 1 μl of restriction enzyme BamHI. After 2 minutes at 37°C, 15 μl of the reaction mixture was removed and mixed with 1 μl of 250 μm EDTA. After 5 minutes at 37°C, the remaining 15 μl of the reaction mixture was also removed and mixed with 1 μl of 250 μM EDTA. This partial BamHI digestion yielded all possible ClaI/BamHI restriction fragments. After electrophoresis on a 0.5% TBE gel, the approximately 12.7 kb ClaI/BamHI restriction fragment was purified from DEAE 81 paper. This restriction fragment comprises the murine variable, human constant gamma-encoding gene. The approximately 12.7 kb ClaI/BamHI (partial) restriction fragment was then ligated into the ClaI/BamHI-digested plasmid pSV2gpt(E-), then transformed into E. coli. After re-isolating and restriction site mapping, the plasmids which comprise the proper restriction maps are designated plasmid pGCEMG(E-).

## D. Construction of Plasmids pNCEMK(E-) and pNCEMG1(E-)

About 10 μg (100 μl) of plasmid pSV2neo(E-) DNA were digested with restriction enzymes ClaI and BamHI, then the vector fragments were isolated and purified in substantial accordance with the teaching of Example 5C. The approximately 9.0 kb ClaI/BamHI restriction fragment of plasmid pHKCE-10 (isolated in Example 5C), which comprises the murine variable, human constant kappa-encoding genes, was then ligated into the ClaI/BamHI digested vector fragment of plasmid pSV2neo(E-) to form plasmid pNCEMK(E-), all in substantial accordance with the teaching of Example 5C. Also in accordance with the teaching of Example 5C, plasmid pNCEMG1(E-) was constructed by ligating the approximately 12.7 kb ClaI/BamHI (partial) restriction fragment of plasmid pHGCE-30 into the ClaI/BamHI digested vector fragment of plasmid pSV2neo(E-). Plasmid pNCEMG1(E-) therefore comprises the murine variable, human constant gamma encoding genes on an SV40 enhancerless expression vector.

## Example 6

### Transfection of Chimeric Light and Heavy Chain Genes with SV40 Enhancerless Expression Vectors

Each of the SV40 enhancerless expression vectors (pGCEMK(E-), pGCEMG(E-), pNCEMK(E-) and pNCEMG1(E-)) were transfected into SP2/0 hybridoma cells by electroporation substantially as described in Example 3A. Each plasmid can be singly transfected, or co-transfected, into the cells, although the best results occur when a vector comprising the kappa chain and the gpt marker are first transfected into the cell, followed by a second transfection of a vector comprising the gamma chain and the neo marker. Following growth on HH2 or any other commercially available media plus 5% FCS followed by selection with the appropriate antibiotic (HMAX for cells containing a gpt vector; G418 for cells containing a ne vector), the cells were assayed for kappa chain secretion in substantial accordance with the teaching of Example 3B. The results of these assays are provided in Table 1.

Table 1

Average Levels of Kappa Chain Production in
SV40 Enhancer Containing and Enhancerless
Clones (SP2/0 Host)

| Plasmid | μg/$10^6$ cells |
|---|---|
| pGCEMK(E-) | 20 |
| pGCEMK | 10 |
| pNCEMK(E-) | 5 |
| pNCEMK | 2 |

Assays were also performed to demonstrate which transfectant cell lines produced the highest levels of total chimeric anti-CEA Antibody. These assays were performed in substantial accordance with the teaching of Example 4. The results of these assays are provided in Table 2.

15

Table 2

Average Levels of Human IgG Production in SV40
Enhancer Containing and Enhancerless Clones
(SP2/0 Host)

| Plasmid | $\mu g/10^6$ cells |
|---|---|
| pNCEMK and pGCEMG1 | 1.8 |
| pGCEMK and pNCEMG1 | 2.0 |
| pNCEMK and pGCEMG(E-) | 0.8 |
| pGCEMK and pNCEMG1(E-) | 1.7 |
| pNCEMK(E-) and pGCEMG1 | 2.8 |
| pGCEMK(E-) and pNCEMG1 | 4.2 |
| pNCEMK(E-) and pGCEMG(E-) | 2.5 |
| pGCEMK(E-) and pNCEMG1(E-) | 3.9 |

## Example 7

### Construction of a High Level Expression System for Expression of Heterologous Immunoglobulin Chains

A. Construction of Plasmid p19HANCH

Intermediate plasmid p19HANCH was first constructed by preparing an oligonucleotide polylinker with the DNA sequence comprising:

```
5'-AATTCAGATCTGGTGACCCGCGGTCGACGGGCTGCAGAATATTGCGGCCGCCATGGATCCA-3'
   |||||||||||||||||||||||||||||||||||||||||||||||||||||||||||||
   3'-GTCTAGACCACTGGGCGCCAGCTGCCCGTCGTCTTATAACGCCGGCGGTACCTAGGTTCGA-5'
```

The linker depicted above was synthesized from single-stranded deoxyoligonucleotides by procedures well known in the art. The single-stranded deoxyoligonucleotides can be synthesized with commercially available instruments, such as the Biosearch 8700 DNA Synthesizer marketed by Biosearch, Inc. (San Raphael, CA.), which utilizes phosphoramidite chemistry. Other procedures for synthesizing DNA are also known in the art. The conventional modified phosphotriester method of synthesizing single stranded DNA is described in Itakura et al., 1977, Science 198:1056 and in Crea et al., 1978, Proc. Nat. Acad. Sci. USA 75:5765. In addition, an especially preferred method of synthesizing DNA is disclosed in Hsiung et al., 1983, Nucleic Acid Research 11:3227 and Narang et al., 1980, Methods in Enzymology 68:90.

About 2.5 μg of each of the two oligonucleotide strands were mixed with 50 μl of 2x Annealing Buffer (0.2 M NaCl, 20 mM Tris-HCl (pH 7.8) and 2 mM EDTA) and 50 μl of water. The reaction mixture was heated to 70°C for 5 minutes then allowed to slowly cool to room temperature so that the two single strands would anneal into one linker segment. Approximately 1 μg of plasmid pUC19 (New England Biolabs) was digested with restriction enzymes EcoRI and HindIII in substantial accordance with earlier teachings. After purification of the about 2.6 kb vector fragment, the synthesized polylinker was ligated into the EcoRI/HindIII digested plasmid pUC19. After transformation into E. coli and re-isolation of plasmid DNA, those plasmids which demonstrated the proper HindIII, SspI, PstI, SstII and EcoRI sites within the linker region were designated plasmid p19HAN.

Plasmid S(-)CHAVL was constructed by first digesting the Bluescript vector, M13(-)SK (Stratagene) with restriction BamHI and SstI, then isolating the large vector fragment in substantial accordance with earlier teaching. Plasmid pMLCH-1 was next digested with restriction enzymes BamHI and SstI, and the approximately 1100 bp fragment which comprises the gene encoding the murine CHA variable region was isolated. Plasmid pMLCH-1 can be conventionally isolated from E. coli K12 HB101/pMLCH-1, a strain deposited and made part of the permanent stock culture collection of the Northern Regional Research Laboratory, 1815 North University Street, Peoria, IL 61604 on November 14, 1988. E. coli K12 HB101/pMLCH-1 is available under the accession number NRRL B-18432. The approximately 1100 bp BamHI/SstI restriction fragment of plasmid pMLCH-1 was then ligated into the BamHI/SstI digested vector M13(-)SK and the plasmid was transformed into E. coli in substantial accordance with prior examples. Following re-isolation and restriction mapping, those plasmids which contained the proper approximately 1100 bp BamHI/SstI fragment were designated plasmid S(-)CHAVL.

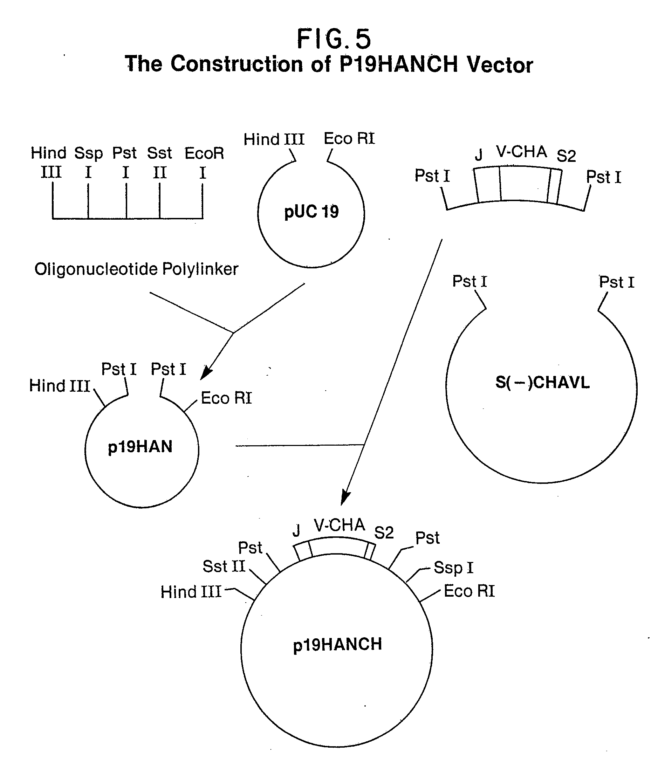

About 1 μg of plasmid S(-)CHAVL was next digested with restriction enzyme PstI in substantial accordance with prior teachings. An approximately 900 bp PstI restriction fragment which comprises the gene encoding the murine CHA variable region was isolated and ligated into plasmid p19HAN, which had first been cut in the polylinker region by restriction enzyme PstI. Following transformation, re-isolation and restriction mapping, those plasmids which comprise the J region of the CHA gene closest to the HindIII site of the polylinker are designated plasmid p19HANCH. A schematic of the construction of plasmid p19HANCH is presented in Figure 5 of the accompanying drawings.

### B. Construction of Expression Vectors pGCHAK-2 and pGCHAK-3

About 5 μg of plasmid pGCEMK (constructed in Example 1) was digested with restriction enzymes ClaI and SspI in substantial accordance with earlier teachings, then the approximately 2.2 kb ClaI/SspI restriction fragment, which comprise the CEM kappa promoter, was purified. This about 2.2 kb ClaI/SspI restriction fragment comprises the CEM kappa promoter. The DNA sequence of the CEM kappa promoter region comprises:

CCCAATATCTGATTTTGATGGCAGCCTGTCATGAGAACATCTATAGACTTGTGGTTTCAGAGC
TTTAAATTGGTCCTTGAGCTTCTATTTTGACTTCCTTCCCAGTGATTACTTCCTGTCTTTGGT
AGTACTTTAGATTGTTTATTTAACCTGGATACTCTCAAACAGCTGTGTAATTTACTTCCTTAT
TTGATGACTATTTTGCATAGATCCCTAGAGCCAGCACAGCTGCCCATGATTTATAAACCATGT
CTTTGCAGTAGATCTAAAATACATCAGACCAGCATGGGCATCAAG

In a separate reaction, plasmid pGCEMK was digested with restriction enzymes ClaI and SstII, and the approximately 9.4 kb vector fragment was isolated. Furthermore, plasmid p19HANCH was digested with restriction enzymes SspI and SstII and the approximately 931 base pair restriction fragment which comprises the gene encoding the murine kappa CHA variable region was isolated. In a three part ligation reaction, the approximately 9.4 kb ClaI/SstII vector fragment of plasmid pGCEMK, the approximately 2.2 kb ClaI/SspI CEM-promotor containing restriction fragment of plasmid pGCEMK and the approximately 931 base pair SspI/SstII restriction fragment of plasmid p19HANCH which contains the gene encoding the murine kappa CHA variable region were all ligated together and transformed into E.coli in substantial accordance with prior examples. After plasmid isolation and restriction mapping, those plasmids which display the proper restriction fragment sizes are esignated plasmid pGCHAK-2.

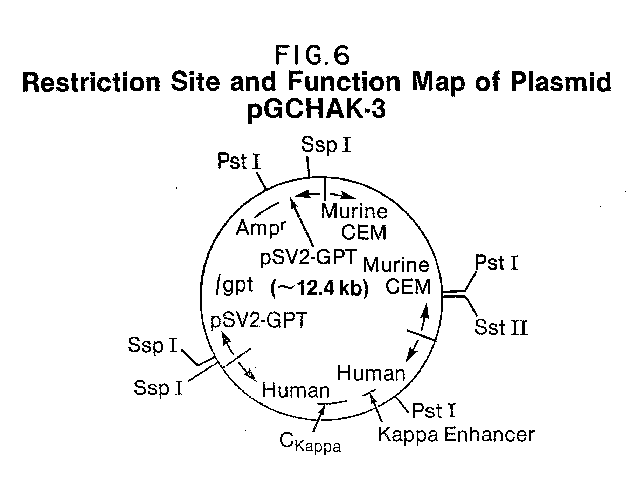

In an analogous manner, plasmid pGCEMK(E-) constructed in Example 5C) was digested with restriction enzymes ClaI and SstII and the approximately 9.2 kb restriction fragment (SV40 enhancerless) was isolated. This fragment was then ligated to the approximately 2.2 kb ClaI/SspI CEM kappa promoter-containing restriction fragment of plasmid pGCEMK and the approximately 931 base pair SspI/SstII restriction fragment of plasmid p19HANCH to form plasmid pGCHAK-3. Plasmid pGCHAK-3 differs from plasmid pGCHAK-2 only in the fact that plasmid pGCHAK-3 lacks the SV40 enhancer. The restriction site and function map of plasmid pGCHAK-3 is presented in Figure 6 of the accompanying drawings.

### C. Construction of Plasmid pNCHAG1

Plasmid pUCVHInc-1A comprises the gene which encodes the murine variable region of heavy metal-binding monoclonal antibody CHA255.5. Plasmid pUCVHInc-1A can be conventionally isolated from E. coli K12 HB101/pUCVHInc-1A, a strain made part of the permanent stock culture collection of the NRRL on November 14, 1988 under accession number NRRL B-18433. Plasmid pHG1Z comprises the human gamma gene and is available from the ATCC under the accession number ATCC 67638. About 1 μg of plasmid pUCVHInc-1A was digested with restriction enzyme HindIII and the approximately 3.4 kb HindIII restriction fragment was isolated. About 1 μg of plasmid pHG1Z was also digested with restriction enzyme HindIII, then treated with Bacterial Alkaline Phosphatase according to procedures well-known in the art. The about 3.4 kb HindIII fragment of plasmid pUCVHInc-1A was then ligated into the HindIII-digested, phosphatased plasmid pHG1Z to form plasmid pHG1-CHA.

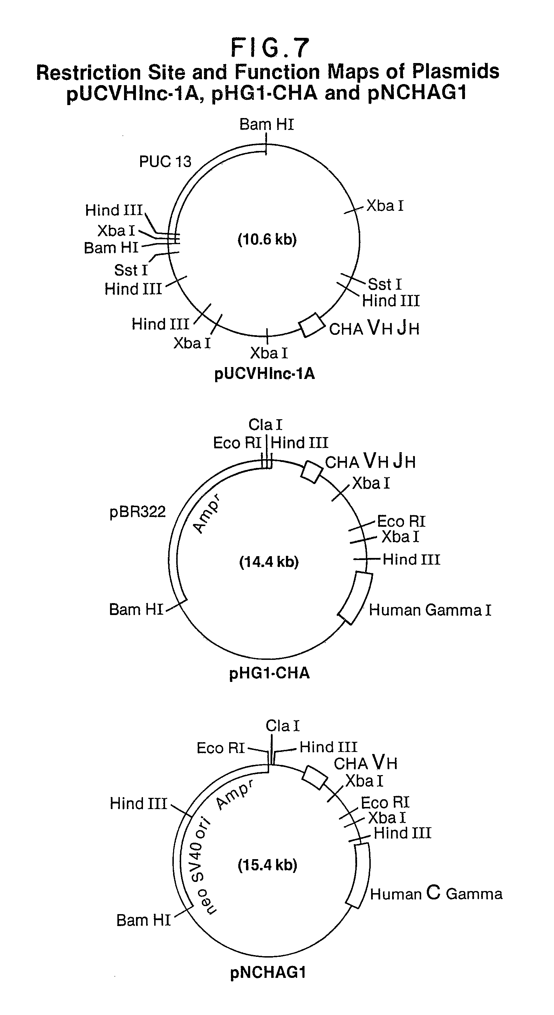

Plasmid pSV2neo-Cla (constructed in Example 1) was digested with restriction enzymes ClaI and BamHI and then about 5.0 kb ClaI/BamHI restriction fragment was isolated. In a likewise manner, plasmid pHG1-CHA was also digested with restriction enzymes ClaI and BamHI and the approximately 10.5 kb restriction fragment was isolated. The about 5.0 kb ClaI/BamHI restriction fragment of plasmid pSV2neo-Cla was ligated to the about 10.5 kb ClaI/BamHI restriction fragment of plasmid pHG1-CHA to form plasmid pNCHAG1. The restriction site and function map of plasmids pUCVHInc-1A, pHG1-CHA and pNCHAG1 are presented in Figure 7 of the accompanying drawings.

### D. Construction of Plasmids pGCHAK and pGCHAK(E-)

About 1 μg of plasmid pMLCH-1 (described in Example 7A) was digested for three minutes with restrictoin enzyme BamHI. After an ethanol precipitation the BamHI ends were made blunt by adding 10 μl of 5 mM each of the four deoxyribonucleotides dTTP, dGTP, dATP and dCTP, two units of Klenow enzyme and 5 μl of 10x

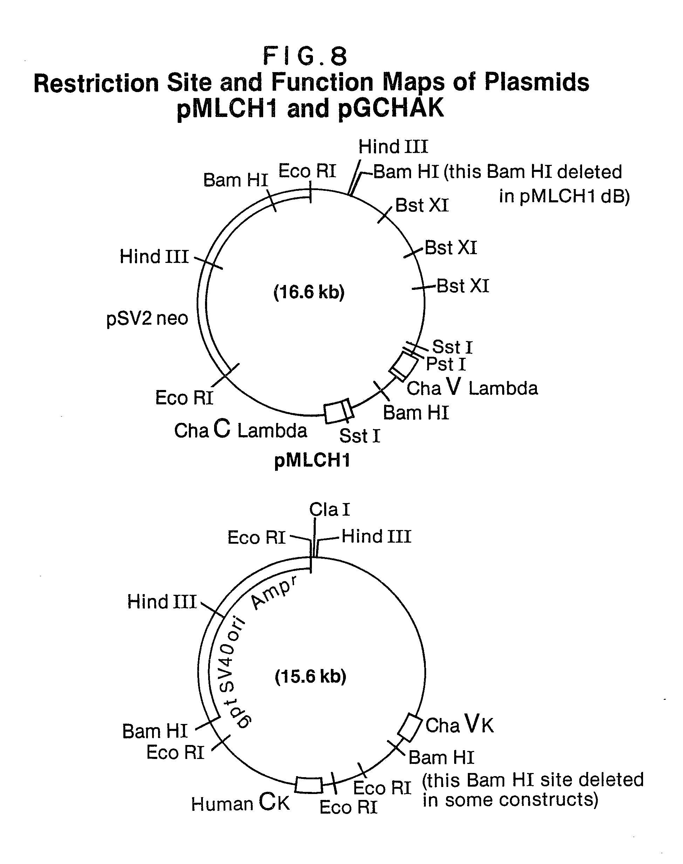

Buffer (.5 M Tris-HCl (pH 7.5), .1 M MgCl$_2$ and 10 mM DTT) in a total of 50 µl reaction volume. After 30 minutes at 37°C, the reaction was stopped by a phenol chloroform extraction and the DNA was self-ligated and transformed into E. coli cells. Those plasmids which demonstrated a deletion of the BamHI site which was 5' of the structural gene were designated plasmid pMLCH1dB. The restriction site and function map of plasmids pMLCH-1 and pMLCH1dB are presented in Figure 8 of the accompanying drawings.

Plasmid pMLCH2 was next constructed by digesting plasmid pMLCH1dB with restriction enzymes BamHI and HindIII and isolating the about 5.75 kb CHA lambda gene region. This fragment was then ligated into BamHI/HindIII digested plasmid pBR322 to form plasmid pMLCH2. Plasmid pMLCH2 was then digested with restriction enzymes ClaI and BamHI and the approximately 5.75 kb restriction fragment was isolated and ligated into the about 4.6 kb ClaI/BamHI digested vector fragment of plasmid pSV2gpt-Cla (constructed in Example 1) to form plasmid pGCHA.

Plasmid pGCHA was digested with restriction enzyme BamHI and the approximately 11.2 kb restriction fragment was isolated. Plasmid pHKF1 (available from the ATCC under the accession number 67637) was digested with restriction enzyme HindIII, filled in with Klenow, phosphorylated BamHI linkers (NEB) were added, then the vector was cut with BamHI and then about 5.2 kb restriction fragment was isolated. This 5.2 kb BamHI fragment of plasmid pHKF-1 was ligated with the about 11.2 kb BamHI fragment of plasmid pGCHA to form plasmid pGCHAK, which comprises the gene which encodes the murine lambda CHA variable region joined to the gene which encodes the human gamma region. The restriction site and function map of plasmid pGCHAK is presented in Figure 8 of the accompanying drawings.

Plasmid pGCHAK(E-) was constructed in substantial accordance with the teaching of the construction of plasmid pGCEMK(E-), using plasmid pSV2gpt-Cla(E-) (constructed in Example 5C).

## Example 8

### Expression of the Heterologous CHA Antibody using the CEM Promoter

The various CHA constructs were transfected into SP2/0 cells in substantial accordance with the teaching of Example 3 and antibody specificity and affinity was measured in substantial accordance with the teaching of Example 4. Initial transfection with plasmid pGCHAK with selection using HMAX followed by a second transfection with plasmid pNCHAG1 with selection on both HMAX and G418 yielded low levels chimeric CHA antibody secretion into the supernatent. Higher levels of antibody production were noted by first transfecting with plasmid pGCHAK-2 followed by transfection with plasmid pNCHAG1. This data demonstrates that the promoter isolated from plasmid pGCEMK is useful to drive high level expression of a heterologous immunoglobulin sequence. Furthermore, transfection with pGCHAK-3 demonstrated a 10-fold higher level of kappa expression than did the transfection with pGCHAK. Therefore, the CEMK promoter from plasmid pGCEMK can best drive the expression of a heterologous immunoglobulin sequence when the initial expression vector lacks the SV40 enhancer.

For teachings concerning the use of certain aspects of this invention in the expression of recombinant bifunctional chimeric antibodies, see M.J. Johnson and J.L. Phelps, Bifunctional chimeric Antibodies, Attorney Docket No. H-7622, U.S. Patent Application No. 07/274,105 filed this even date, herein incorporated by reference (European Patent Application No. 89302313.5

## Claims

1. A DNA construct comprising a first DNA sequence which encodes for the light chain variable region of a chimeric monoclonal antibody, the first DNA sequence coding for an amino acid sequence substantially the same as:

Asp - Ile - Val - Met - Thr - Gln - Ser - Gln - Lys
Phe - Met - Ser - Thr - Ser - Val - Gly - Asp - Arg
Val - Ser - Ile - Thr - Cys - Lys - Ala - Ser - Gln
Asn - Val - Arg - Thr - Ala - Val - Ala - Trp - Tyr
Gln - Gln - Lys - Pro - Gly - Gln - Ser - Pro - Lys
Ala - Leu - Ile - Tyr - Leu - Ala - Ser - Asn - Arg
Tyr - Thr - Gly - Val - Pro - Asp - Arg - Phe - Thr
Gly - Ser - Gly - Ser - Gly - Thr - Asp - Phe - Thr
Leu - Thr - Gle - Thr - Asn - Val - Gln - Ser - Glu
Asp - Leu - Ala - Asp - Tyr - Phe - Cys - Leu - Gln
His - Trp - Asn - Tyr - Pro - Leu - Thr - Phe - Gly
Ala - Gly - Thr - Lys - Leu - Glu - Leu - Lys - Arg.

2. The DNA construct according to Claim 1 wherein the first DNA strand coding sequence is substantially the same as:

GAC - ATT - GTG - ATG - ACC - CAG - TCT - CAA - AAA
TTC - ATG - TCC - ACA - TCA - GTA - GGA - GAC - AGG

GTC - AGC - ATC - ACC - TGC - AAG - GCC - AGT - CAG
AAT - GTT - CGT - ACT - GCT - GTT - GCC - TGG - TAT
CAA - CAG - AAA - CCA - GGG - CAG - TCT - CCT - AAA
GCA - CTG - ATT - TAC - TTG - GCA - TCC - AAC - CGG
TAC - ACT - GGA - GTC - CCT - GAT - CGC - TTC - ACA
GGC - AGT - GGA - TCT - GGG - ACA - GAT - TTC - ACT
CTC - ACC - ATT - ACC - AAT - GTG - CAA - TCT - GAA
GAC - CTG - GCA - GAT - TAT - TTC - TGT - CTG - CAA
CAT - TGG - AAT - TAT - CCG - CTC - ACG - TTC - GGT
GCT - GGG - ACC - AAG - CTG - GAG - CTG - AAA - CGG.

3. The DNA construct according to Claim 1 or 2 wherein the first DNA strand coding sequence further comprises a DNA sequence encoding for a eukaryotic leader peptide.

4. The DNA construct according to Claim 1, 2 or 3 wherein the DNA construct further comprises a second DNA strand sequence which encodes for the light chain constant region of the chimeric monoclonal antibody.

5. The DNA construct according to Claim 3 wherein the DNA strand sequence encoding for the leader peptide is substantially the same as:

ATG - GAG - TTT - CAG - ACC - CAG - GTC - TTT - GTA
TTC - GTG - TTG - CTC - TGG - TTG - TCT - GGT - GTT
GAT - GGA.

6. A DNA construct comprising a first DNA sequence which encodes for the heavy chain variable region of a chimeric monoclonal antibody, the first DNA sequence coding for an amino acid sequence substantially the same as:

Asp - Val - Gln - Leu - Val - Glu - Ser - Gly - Gly
Gly - Leu - Val - Gln - Pro - Gly - Gly - Ser - Arg
Lys - Leu - Ser - Cys - Ala - Ala - Ser - Gly - Phe
Thr - Phe - Ser - Asn - Phe - Gly - Met - His - Trp
Ile - Arg - Gln - Ala - Pro - Glu - Lys - Gly - Leu
Glu - Trp - Val - Ala - Tyr - Ile - Ser - Gly - Gly
Ser - Ser - Thr - Ile - Tyr - Tyr - Ala - Asp - Thr
Val - Lys - Gly - Arg - Phe - Thr - Ile - Ser - Arg
Asp - Asn - Pro - Arg - Asn - Thr - Leu - Phe - Leu
Gln - Met - Thr - Ser - Leu - Arg - Ser - Glu - Asp
Thr - Ala - Met - Phe - Tyr - Cys - Ala - Arg - Asp
Tyr - Tyr - Ala - Asn - Asn - Tyr - Trp - Tyr - Phe
Asp - Val - Trp - Gly - Ala - Gly - Thr - Thr - Val
Thr - Val - Ser - Ser - Ala.

7. The DNA construct according to Claim 6 wherein the first DNA strand coding sequence is substantially the same as:

GAT - GTG - CAG - CTG - GTG - GAG - TCT - GGG - GGA
GGC - TTA - GTG - CAG - CCT - GGA - GGG - TCC - CGG
AAA - CTC - TCC - TGT - GCA - GCC - TCT - GGA - TTC
ACT - TTC - AGT - AAC - TTT - GGA - ATG - CAC - TGG
ATT - CGT - CAG - GCT - CCA - GAG - AAG - GGA - CTG
GAG - TGG - GTC - GCA - TAC - ATT - AGT - GGT - GGC
AGT - AGT - ACC - ATC - TAC - TAT - GCA - GAC - ACA
GTG - AAG - GGC - CGA - TTC - ACC - ATC - TCC - AGA
GAG - AAT - CCC - AGG - AAC - ACC - CTC - TTC - CTG
CAA - ATG - ACC - AGT - CTA - AGG - TCT - GAG - GAC
ACC - GCC - ATG - TTT - TAC - TGT - GCA - AGA - GAT
TAC - TAC - GCT - AAC - AAC - TAC - TGG - TAC - TTC
GAT - GTC - TGG - GGC - GCA - GGG - ACC - ACG - GTC
ACC - GTC - TCC - TCA - GCC.

8. The DNA construct according to Claims 6 or 7 wherein the DNA construct further comprises a second DNA strand sequence which encodes for the heavy chain constant region of the chimeric monoclonal antibody.

9. The DNA construct according to Claim 6 wherein the first DNA strand coding sequences further comprises a DNA sequence encoding for a eukaryotic leader peptide.

10. The DNA construct according to Claim 9 wherein the DNA strand sequence encoding for the leader peptide is substantially the same as:

ATG - GAC - TCC - AGG - CTC - AAT - TTA - GTT - TTC - CTT
GTC - CTT - ATT - TTA - AAA - GGT - GTC - CAG - TGT.

11. The DNA construct according to any of Claims 1 to 10 wherein the first DNA strand coding sequence is derived from a murine hybridoma.

12. The DNA construct according to Claim 11 wherein the murine hybridoma is CEM 231.6.7.

19

13. The DNA construct according to Claim 4 or 8 wherein the second DNA strand coding sequence is derived from a human lymphocyte.

14. A eukaryotic host cell capable of expression of a chimeric monoclonal antibody comprising the DNA construct of any of Claims 1 to 5 encoding for the light chain of the chimeric antibody, and transcriptional and translational DNA sequences positioned in relation to the light chain-encoding DNA sequence to direct expression of the light chain.

15. The eukaryotic host cell according to Claim 17 wherein a second DNA construct comprises, or the first DNA construct further comprises, the construct of any of Claims 6 to 10 encoding for the heavy chain of the chimeric antibody, and transcriptional and translational DNA sequences positioned in relation to the heavy chain-encoding DNA strand sequence to direct expression of the heavy chain.

16. A chimeric monoclonal antibody comprising a light chain variable region having an amino acid sequence substantially the same as:

Asp - Ile - Val - Met - Thr - Gln - Ser - Gln - Lys
Phe - Met - Ser - Thr - Ser - Val - Gly - Asp - Arg
Val - Ser - Ile - Thr - Cys - Lys - Ala - Ser - Gln
Asn - Val - Arg - Thr - Ala - Val - Ala - Trp - Tyr
Gln - Gln - Lys - Pro - Gly - Gln - Ser - Pro - Lys
Ala - Leu - Ile - Tyr - Leu - Ala - Ser - Asn - Arg
Tyr - Thr - Gly - Val - Pro - Asp - Arg - Phe - Thr
Gly - Ser - Gly - Ser - Gly - Thr - Asp - Phe - Thr
Leu - Thr - Ile - Thr - Asn - Val - Gln - Ser - Glu
Asp - Leu - Ala - Asp - Tyr - Phe - Cys - Leu - Gln
His - Trp - Asn - Tyr - Pro - Leu - Thr - Phe - Gly
Ala - Gly - Thr - Lys - Leu - Glu - Leu - Lys - Arg.

17. A chimeric monoclonal antibody comprising a heavy chain variable region having an amino acid sequence substantially the same as:

Asp - Val - Gln - Leu - Val - Glu - Ser - Gly - Gly
Gly - Leu - Val - Gln - Pro - Gly - Gly - Ser - Arg
Lys - Leu - Ser - Cys - Ala - Ala - Ser - Gly - Phe
Thr - Phe - Ser - Asn - Phe - Gly - Met - His - Trp
Ile - Arg - Gln - Ala - Pro - Glu - Lys - Gly - Leu
Glu - Trp - Val - Ala - Tyr - Ile - Ser - Gly - Gly
Ser - Ser - Thr - Ile - Tyr - Tyr - Ala - Asp - Thr
Val - Lys - Gly - Arg - Phe - Thr - Ile - Ser - Arg
Asp - Asn - Pro - Arg - Asn - Thr - Leu - Phe - Leu
Gln - Met - Thr - Ser - Leu - Arg - Ser - Glu - Asp
Thr - Ala - Met - Phe - Tyr - Cys - Ala - Arg - Asp
Tyr - Tyr - Ala - Asn - Asn - Tyr - Trp - Tyr - Phe
Asp - Val - Trp - Gly - Ala - Gly - Thr - Thr - Val
Thr - Val - Ser - Ser - Ala

18. A chimeric monoclonal antibody comprising a light chain variable region having the amino acid sequence of Claim 16 and a heavy chain variable region having the amino acid sequence of Claim 17.

19. The chimeric monoclonal antibody according to Claim 16, 17 or 18 wherein the variable region of the chain is derived from a murine hybridoma.

20. The chimeric monoclonal antibody according to Claim 19 wherein the murine hybridoma is CEM 231.6.7.

21. The chimeric monoclonal antibody according to any of Claims 16 to 20 wherein the constant region of the antibody is derived from a human lymphocyte.

22. The chimeric monoclonal antibody according to any of Claims 16 to 21 wherein the antibody is XCEM 449.

23. The DNA construct of Claim 1 or 2 that is plasmid pMLCE-10 as shown in Figure 1 or plasmid pHKCE-10 as shown in Figure 2.

24. The DNA construct of Claim 4 that is plasmid pGCEMK as shown in Figure 2.

25. The DNA construct of Claim 6 that is plasmid pMHCE-30 as shown in Figure 3 or pHGCEM-30 as shown in Figure 4.

26. The DNA construct of Claim 10 that is plasmid pNCEMG1 as shown in Figure 4.

27. A method of increasing the expression of chimeric antibodies in transfected cells, said method comprising the steps of:

a) constructing an SV40 vector-based recombinant DNA expression vector which comprises one or more genes encoding immunoglobulin chains, said vector lacking the SV40 enhancer,

b) transfecting said vector into a mammalian host cell, and

c) culturing said host cell under conditions suitable for the expression of said immunoglobulin genes.

28. The method of Claim 27 wherein the enhancerless vector is plasmid pGCEMK(E-) as described in Example 5C, plasmid pNCEMK(E-) as described in Example 5D, plasmid pGCEMG1(E-) as described in Example 5C, or plasmid pNCEMG1(E-) as described in Example 5D.

29. Plasmid pGCEMK(E-), plasmid pNCEMK(E-), plasmid pGCEMG1(E-) or, plasmid pNCEMG1(E-).

30. The approximately 2.2 kilobase ClaI/SspI CEM kappa promoter-containing restriction fragment of plasmid pGCEMK.

31. The CEM kappa promoter of plasmid pGCEMK.

32. The CEM kappa promoter of Claim 31 wherein the nucleotide sequence comprises:
CCCAATATCTGATTTTGATGGCAGCCTGTCATGAGAACATCTATAGACTTGTGGTTTCAGAGC
TTTAAATTGGTCCTTGAGCTTCTATTTTGACTTCCTTCCCAGTGATTACTTCCTGTCTTTGGT
AGTACTTTAGATTGTTTATTTAACCTGGATACTCTCAAACAGCTGTGTAATTTACTTCCTTAT
TTGATGACTATTTTGCATAGATCCCTAGAGCCAGCACAGCTGCCCATGATTTATAAACCATGT
CTTTGCAGTAGATCTAAAATACATCAGACCAGCATGGGCATCAAG

## Claims for the following Contracting State: GR

1. A DNA construct comprising a first DNA sequence which encodes for the light chain variable region of a chimeric monoclonal antibody, the first DNA sequence coding for an amino acid sequence substantially the same as:
Asp - Ile - Val - Met - Thr - Gln - Ser - Gln - Lys
Phe - Met - Ser - Thr - Ser - Val - Gly - Asp - Arg
Val - Ser - Ile - Thr - Cys - Lys - Ala - Ser - Gln
Asn - Val - Arg - Thr - Ala - Val - Ala - Trp - Tyr
Gln - Gln - Lys - Pro - Gly - Gln - Ser - Pro - Lys
Ala - Leu - Ile - Tyr - Leu - Ala - Ser - Asn - Arg
Tyr - Thr - Gly - Val - Pro - Asp - Arg - Phe - Thr
Gly - Ser - Gly - Ser - Gly - Thr - Asp - Phe - Thr
Leu - Thr - Ile - Thr - Asn - Val - Gln - Ser - Glu
Asp - Leu - Ala - Asp - Tyr - Phe - Cys - Leu - Gln
His - Trp - Asn - Tyr - Pro - Leu - Thr - Phe - Gly
Ala - Gly - Thr - Lys - Leu - Glu - Leu - Lys - Arg.

2. The DNA construct according to Claim 1 wherein the first DNA strand coding sequence is substantially the same as:
GAC - ATT - GTG - ATG - ACC - CAG - TCT - CAA - AAA
TTC - ATG - TCC - ACA - TCA - GTA - GGA - GAC - AGG
GTC - AGC - ATC - ACC - TGC - AAG - GCC - AGT - CAG
AAT - GTT - CGT - ACT - GCT - GTT - GCC - TGG - TAT
CAA - CAG - AAA - CCA - GGG - CAG - TCT - CCT - AAA
GCA - CTG - ATT - TAC - TTG - GCA - TCC - AAC - CGG
TAC - ACT - GGA - GTC - CCT - GAT - CGC - TTC - ACA
GGC - AGT - GGA - TCT - GGG - ACA - GAT - TTC - ACT
CTC - ACC - ATT - ACC - AAT - GTG - CAA - TCT - GAA
GAC - CTG - GCA - GAT - TAT - TTC - TGT - CTG - CAA
CAT - TGG - AAT - TAT - CCG - CTC - ACG - TTC - GGT
GCT - GGG - ACC - AAG - CTG - GAG - CTG - AAA - CGG.

3. The DNA construct according to Claim 1 or 2 wherein the first DNA strand coding sequence further comprises a DNA sequence encoding for a eukaryotic leader peptide.

4. The DNA construct according to Claim 1, 2 or 3 wherein the DNA construct further comprises a second DNA strand sequence which encodes for the light chain constant region of the chimeric monoclonal antibody.

5. The DNA construct according to Claim 3 wherein the DNA strand sequence encoding for the leader peptide is substantially the same as:
ATG - GAG - TTT - CAG - ACC - CAG - GTC - TTT - GTA
TTC - GTG - TTG - CTC - TGG - TTG - TCT - GGT - GTT
GAT - GGA.

6. A DNA construct comprising a first DNA sequence which encodes for the heavy chain variable region of a chimeric monoclonal antibody, the first DNA sequence coding for an amino acid sequence substantially the same as:
Asp - Val - Gln - Leu - Val - Glu - Ser - Gly - Gly
Gly - Leu - Val - Gln - Pro - Gly - Gly - Ser - Arg
Lys - Leu - Ser - Cys - Ala - Ala - Ser - Gly - Phe
Thr - Phe - Ser - Asn - Phe - Gly - Met - His - Trp
Ile - Arg - Gln - Ala - Pro - Glu - Lys - Gly - Leu
Glu - Trp - Val - Ala - Tyr - Ile - Ser - Gly - Gly
Ser - Ser - Thr - Ile - Tyr - Tyr - Ala - Asp - Thr
Val - Lys - Gly - Arg - Phe - Thr - Ile - Ser - Arg
Asp - Asn - Pro - Arg - Asn - Thr - Leu - Phe - Leu

Gln - Met - Thr - Ser - Leu - Arg - Ser - Glu - Asp
Thr - Ala - Met - Phe - Tyr - Cys - Ala - Arg - Asp
Tyr - Tyr - Ala - Asn - Asn - Tyr - Trp - Tyr - Phe
Asp - Val - Trp - Gly - Ala - Gly - Thr - Thr - Val
Thr - Val - Ser - Ser - Ala.

7. The DNA construct according to Claim 6 wherein the first DNA strand coding sequence is substantially the same as:

GAT - GTG - CAG - CTG - GTG - GAG - TCT - GGG - GGA
GGC - TTA - GTG - CAG - CCT - GGA - GGG - TCC - CGG
AAA - CTC - TCC - TGT - GCA - GCC - TCT - GGA - TTC
ACT - TTC - AGT - AAC - TTT - GGA - ATG - CAC - TGG
ATT - CGT - CAG - GCT - CCA - GAG - AAG - GGA - CTG
GAG - TGG - GTC - GCA - TAC - ATT - AGT - GGT - GGC
AGT - AGT - ACC - ATC - TAC - TAT - GCA - GAC - ACA
GTG - AAG - GGC - CGA - TTC - ACC - ATC - TCC - AGA
GAC - AAT - CCC - AGG - AAC - ACC - CTC - TTC - CTG
CAA - ATG - ACC - AGT - CTA - AGG - TCT - GAG - GAC
ACG - GCC - ATG - TTT - TAC - TGT - GCA - AGA - GAT
TAC - TAC - GCT - AAC - AAC - TAC - TGG - TAC - TTC
GAT - GTC - TGG - GGC - GCA - GGG - ACC - ACG - GTC
ACC - GTC - TCC - TCA - GCC.

8. The DNA construct according to Claims 6 or 7 wherein the DNA construct further comprises a second DNA strand sequence which encodes for the heavy chain constant region of the chimeric monoclonal antibody.

9. The DNA construct according to Claim 6 wherein the first DNA strand coding sequences further comprises a DNA sequence encoding for a eukaryotic leader peptide.

10. The DNA construct according to Claim 9 wherein the DNA strand sequence encoding for the leader peptide is substantially the same as:

ATG - GAC - TCC - AGG - CTC - AAT - TTA - GTT - TTC - CTT
GTC - CTT - ATT - TTA - AAA - GGT - GTC - CAG - TGT.

11. The DNA construct according to any of Claims 1 to 10 wherein the first DNA strand coding sequence is derived from a murine hybridoma.

12. The DNA construct according to Claim 11 wherein the murine hybridoma is CEM 231.6.7.

13. The DNA construct according to Claim 4 or 8 wherein the second DNA strand coding sequence is derived from a human lymphocyte.

14. A eukaryotic host cell capable of expression of a chimeric monoclonal antibody comprising the DNA construct of any of Claims 1 to 5 encoding for the light chain of the chimeric antibody, and transcriptional and translational DNA sequences positioned in relation to the light chain-encoding DNA sequence to direct expression of the light chain.

15. The eukaryotic host cell according to Claim 17 wherein a second DNA construct comprises, or the first DNA construct further comprises, the construct of any of Claims 6 to 10 encoding for the heavy chain of the chimeric antibody, and transcriptional and translational DNA sequences positioned in relation to the heavy chain-encoding DNA strand sequence to direct expression of the heavy chain.

16. The DNA construct of Claim 1 or 2 that is plasmid pMLCE-10 as shown in Figure 1 or plasmid pHKCE-10 as shown in Figure 2.

17. The DNA construct of Claim 4 that is plasmid pGCEMK as shown in Figure 2.

18. The DNA construct of Claim 6 that is plasmid pMHCE-30 as shown in Figure 3 or pHGCEM-30 as shown in Figure 4.

19. The DNA construct of Claim 10 that is plasmid pNCEMG1 as shown in Figure 4.

20. A method of expressing chimeric antibodies which comprise the variable region of Claim 1 or 6 in transfected cells, said method comprising the steps of:

a) constructing a recombinant DNA vector which comprises one or more genes encoding the variable region of Claim 1 or 6,

b) transfecting said vector into a mammalian host cell, and

c) culturing said host cell under conditions suitable for the expression of said variable region genes.

21. The method of Claim 20 wherein the DNA construct is plasmid pGCEMK or pNCEMG1.

22. A method of increasing the expression of chimeric antibodies in transfected cells, said method comprising the steps of:

a) constructing an SV40 vector-based recombinant DNA expression vector which comprises one or more genes encoding immunoglobulin chains, said vector lacking the SV40 enhancer,

b) transfecting said vector into a mammalian host cell, and

c) culturing said host cell under conditions suitable for the expression of said immunoglobulin genes.

23. The method of Claim 27 wherein the enhancerless vector is plasmid pGCEMK(E-) as described in

22

Example 5C, plasmid pNCEMK(E-) as described in Example 5D, plasmid pGCEMG1(E-) as described in Example 5C, or plasmid pNCEMG1(E-) as described in Example 5D.

24. Plasmid pGCEMK(E-), plasmid pNCEMK(E-), plasmid pGCEMG1(E-) or, plasmid pNCEMG1(E-).

25. The approximately 2.2 kilobase ClaI/SspI CEM kappa promoter-containing restriction fragment of plasmid pGCEMK.

26. The CEM kappa promoter of plasmid pGCEMK.

27. The CEM kappa promoter of Claim 26 wherein the nucleotide sequence comprises:
CCCAATATCTGATTTTGATGGCAGCCTGTCATGAGAACATCTATAGACTTGTGGTTTCAGAGC
TTTAAATTGGTCCTTGAGCTTCTATTTTGACTTCCTTCCCAGTGATTACTTCCTGTCTTTGGT
AGTACTTTAGATTGTTTATTTAACCTGGATACTCTCAAACAGCTGTGTAATTTACTTCCTTAT
TTGATGACTATTTTGCATAGATCCCTAGAGCCAGCACAGCTGCCCATGATTTATAAACCATGT
CTTTGCAGTAGATCTAAAATACATCAGACCAGCATGGGCATCAAG

## Claims for the following Contracting State: ES

1. A method of expressing chimeric antibody chains in transfected cells, said antibody chains comprising variable regions which bind to human carcenoembryonic antigen, said method comprising the steps of:

a) constructing a recombinant DNA vector which comprises a gene encoding a light chain variable region with the amino acid sequence:
Asp - Ile - Val - Met - Thr - Gln - Ser - Gln - Lys
Phe - Met - Ser - Thr - Ser - Val - Gly - Asp - Arg
Val - Ser - Ile - Thr - Cys - Lys - Ala - Ser - Gln
Asn - Val - Arg - Thr - Ala - Val - Ala - Trp - Tyr
Gln - Gln - Lys - Pro - Gly - Gln - Ser - Pro - Lys
Ala - Leu - Ile - Tyr - Leu - Ala - Ser - Asn - Arg
Tyr - Thr - Gly - Val - Pro - Asp - Arg - Phe - Thr
Gly - Ser - Gly - Ser - Gly - Thr - Asp - Phe - Thr
Leu - Thr - Ile - Thr - Asn - Val - Gln - Ser - Glu
Asp - Leu - Ala - Asp - Tyr - Phe - Cys - Leu - Gln
His - Trp - Asn - Tyr - Pro - Leu - Thr - Phe - Gly
Ala - Gly - Thr - Lys - Leu - Glu - Leu - Lys - Arg
b) transfecting said vector into a mammalian host cell, and
c) culturing said host cell under conditions suitable for the expression of said variable region gene.

2. The method of Claim 1 wherein the recombinant DNA vector is plasmid pGCEMK as described in Example 1Q.

3. The method of Claim 2 wherein the transfected cell is an SP2/0 cell.

4. A method of expressing chimeric antibody chains in transfected cells, said antibody chains comprising variable regions which bind to human carcinoembryonic antigen, said method comprising the steps of:

a) constructing a recombinant DNA vector which comprises a gene encoding a heavy chain variable region with the amino acid sequence:
Asp - Val - Gln - Leu - Val - Glu - Ser - Gly - Gly
Gly - Leu - Val - Gln - Pro - Gly - Gly - Ser - Arg
Lys - Leu - Ser - Cys - Ala - Ala - Ser - Gly - Phe
Thr - Phe - Ser - Asn - Phe - Gly - Met - His - Trp
Ile - Arg - Gln - Ala - Pro - Glu - Lys - Gly - Leu
Glu - Trp - Val - Ala - Tyr - Ile - Ser - Gly - Gly
Ser - Ser - Thr - Ile - Tyr - Tyr - Ala - Asp - Thr
Val - Lys - Gly - Arg - Phe - Thr - Ile - Ser - Arg
Asp - Asn - Pro - Arg - Asn - Thr - Leu - Phe - Leu
Gln - Met - Thr - Ser - Leu - Arg - Ser - Glu - Asp
Thr - Ala - Met - Phe - Tyr - Cys - Ala - Arg - Asp
Tyr - Tyr - Ala - Asn - Asn - Tyr - Trp - Tyr - Phe
Asp - Val - Trp - Gly - Ala - Gly - Thr - Thr - Val
Thr - Val - Ser - Ser - Ala,
b) transfecting said vector into a mammalian host cell, and
c) culturing said host cell under conditions suitable for the expression of said variable region.

5. The method of Claim 3 wherein the recombinant DNA vector is plasmid pNCEMG1 as described in Example 1R.

6. The method of Claim 2 wherein the transfected cell is an SP2/0 cell.

7. A method of expressing chimeric antibodies in transfected cells, said antibodies comprising variable regions which bind to human carcinoembryonic antigen, said method comprising the steps of:

a) constructing a recombinant DNA encoding a light chain variable region with the amino acid sequence:

Asp - Ile - Val - Met - Thr - Gln - Ser - Gln - Lys
Phe - Met - Ser - Thr - Ser - Val - Gly - Asp - Arg
Val - Ser - Ile - Thr - Cys - Lys - Ala - Ser - Gln
Asn - Val - Arg - Thr - Ala - Val - Ala - Trp - Tyr
Gln - Gln - Lys - Pro - Gly - Gln - Ser - Pro - Lys
Ala - Leu - Ile - Tyr - Leu - Ala - Ser - Asn - Arg
Tyr - Thr - Gly - Val - Pro - Asp - Arg - Phe - Thr
Gly - Ser - Gly - Ser - Gly - Thr - Asp - Phe - Thr
Leu - Thr - Ile - Thr - Asn - Val - Gln - Ser - Glu
Asp - Leu - Ala - Asp - Tyr - Phe - Cys - Leu - Gln
His - Trp - Asn - Tyr - Pro - Leu - Thr - Phe - Gly
Ala - Gly - Thr - Lys - Leu - Glu - Leu - Lys - Arg
and

b) constructing a recombinant DNA vector which comprises a gene encoding a heavy chain variable region with the amino sequence:
Asp - Val - Gln - Leu - Val - Glu - Ser - Gly - Gly
Gly - Leu - Val - Gln - Pro - Gly - Gly - Ser - Arg
Lys - Leu - Ser - Cys - Ala - Ala - Ser - Gly - Phe
Thr - Phe - Ser - Asn - Phe - Gly - Met - His - Trp
Ile - Arg - Gln - Ala - Pro - Glu - Lys - Gly - Leu
Glu - Trp - Val - Ala - Tyr - Ile - Ser - Gly - Gly
Ser - Ser - Thr - Ile - Tyr - Tyr - Ala - Asp - Thr
Val - Lys - Gly - Arg - Phe - Thr - Ile - Ser - Arg
Asp - Asn - Pro - Arg - Asn - Thr - Leu - Phe - Leu
Gln - Met - Thr - Ser - Leu - Arg - Ser - Glu - Asp
Thr - Ala - Met - Phe - Tyr - Cys - Ala - Arg - Asp
Tyr - Tyr - Ala - Asn - Asn - Tyr - Trp - Tyr - Phe
Asp - Val - Trp - Gly - Ala - Gly - Thr - Thr - Val
Thr - Val - Ser - Ser - Ala,
or

c) constructing a recombinant DNA vector which comprises said gene encoding a light chain variable region and said gene encoding a heavy chain variable region, and

d) transfecting said vectors a) and b) or said vector c) into a mammalian host cell, and

e) culturing said host cell under conditions suitable for the expression of said variable region genes.

8. The method of Claim 7 wherein the plasmid of a) is plasmid pGCEMK and the plasmid of b) is plasmid pNCEMG1.

9. The method of Claim 8 wherein the transfected cell is an SP2/0 cell.

10. A method of increasing the expression of chimeric antibodies in transfected cells, said method comprising the steps of:

a) constructing an SV40 vector-based recombinant DNA expression vector which comprises one or more genes encoding immunoglobulin chains, said vector lacking the SV40 enhancer,

b) transfecting said vector into a mammalian host cell, and

c) culturing said host cell under conditions suitable for the expression of said immunoglobulin genes.

11. The method of Claim 10 wherein the enhancerless vector is plasmid pGCEMK(E-) as described in Example 5C, plasmid pNCEMK(E-) as described in Example 5D, plasmid pGCEMG1(E-) as described in Example 5C, or plasmid pNCEMG1(E-) as described in Example 5D.

12. The method of Claim 11 wherein the transfected host cell is an SP2/0 cell.

13. A method of increasing the expression of chimeric antibodies in transfected cells, said method comprising the steps of:

a) constructing a recombinant DNA vector which comprises the CEM Kappa promoter of plasmid pGCEMK as described in Example 7B and which further comprises one or more genes encoding immunoglobulin chains,

b) transfecting said vector into a mammalian host cell, and

c) culturing said host cell under conditions suitable for the expression of said immunoglobulin genes.

14. The method of Claim 13 wherein the recombinant DNA vector is plasmid pGCHAK-2 as described in Example 7B or plasmid pGCHAK-3 as described in Example 7B.

15. The method of Claim 14 wherein the transfected host cell is an SP2/0 cell.

# FIG.I
# Restriction Site and Function Maps of Plasmids
# pMLCE-10 and pHKF-1

pMLCE-10

pHKF-1

# FIG.2
# Restriction Site and Function Maps of Plasmids pHKCe-10 and pGCEMK

# FIG.3
# Restriction Site and Function Maps of Plasmids pMHCE-30 and pHG1Z

pMHCE-30

pHG1Z

# FIG. 4
## Restriction Site and Function Maps of Plasmids pHGCEM-30 and pNCEMG1

pHGCEM-30

pNCEMG1

# FIG. 5
## The Construction of P19HANCH Vector

# FIG.6
## Restriction Site and Function Map of Plasmid pGCHAK-3

# FIG.7
## Restriction Site and Function Maps of Plasmids pUCVHInc-1A, pHG1-CHA and pNCHAG1

pUCVHInc-1A

pHG1-CHA

pNCHAG1

# F I G. 8
## Restriction Site and Function Maps of Plasmids pMLCH1 and pGCHAK

pMLCH1

pGCHAK